# EUROPÄISCHE PATENTANMELDUNG

(11) **EP 4 600 337 A2**
(43) Veröffentlichungstag der Anmeldung: **13.08.2025**
(21) Anmeldenummer: 25181098.2
(22) Anmeldetag: 01.06.2017
(51) Int. Cl.: C11D 3/386

(54) **BACILLUS GIBSONII PROTEASE UND VARIANTEN DAVON**

(30) Priorität: 15.06.2016 DE 102016210628
(62) Teilanmeldung aus: 17728501.2
(71) Anmelder: Henkel AG & Co. KGaA, 40589 Düsseldorf (DE)
(72) Erfinder: Herbst, Daniela, 01309 Dresden (DE); O'Connell, Timothy, 86899 Landsberg am Lech (DE); Mussmann, Nina, 47877 Willich (DE); Vockenroth, Inga Kerstin, 40227 Düsseldorf (DE); Weber, Thomas, 35096 Weimar (Lahn) (DE)

(57) **Zusammenfassung**

Die Erfindung betrifft Proteasen umfassend eine Aminosäuresequenz, die mindestens 70% Sequenzidentität mit der in SEQ ID NO:1 angegebenen Aminosäuresequenz über deren Gesamtlänge aufweist und die eine Aminosäuresubstitution an mindestens einer der Positionen, die den Positionen 12, 43, 122, 127, 154, 156, 160, 211, 212 oder 222, jeweils bezogen auf die Nummerierung gemäß SEQ ID NO:1, entsprechen, aufweisen sowie deren Herstellung und Verwendung. Derartige Proteasen zeigen eine sehr gute Reinigungsleistung.

## Beschreibung

Die Erfindung liegt auf dem Gebiet der Enzymtechnologie. Die Erfindung betrifft Proteasen aus *Bacillus gibsonii* deren Aminosäuresequenz insbesondere im Hinblick auf den Einsatz in Wasch- und Reinigungsmitteln verändert wurden, um ihnen eine bessere Reinigungsleistung bei der Entfernung von Protein-haltigen Anschmutzungen, insbesondere gegenüber eingetrockneten oder eingebrannten Anschmutzungen, zu verleihen, und die für sie codierende Nukleinsäuren sowie deren Herstellung. Die Erfindung betrifft ferner die Verwendungen dieser Proteasen und Verfahren, in denen sie eingesetzt werden, sowie diese enthaltende Mittel, insbesondere Wasch- und Reinigungsmittel.

Proteasen gehören zu den technisch bedeutendsten Enzymen überhaupt. Für Wasch- und Reinigungsmittel sind sie die am längsten etablierten und in praktisch allen modernen, leistungsfähigen Wasch- und Reinigungsmitteln enthaltenen Enzyme. Sie bewirken den Abbau proteinhaltiger Anschmutzungen auf dem Reinigungsgut. Hierunter sind wiederum Proteasen vom Subtilisin-Typ (Subtilasen, Subtilopeptidasen, EC 3.4.21.62) besonders wichtig, welche aufgrund der katalytisch wirksamen Aminosäuren Serin-Proteasen sind. Sie wirken als unspezifische Endopeptidasen und hydrolysieren beliebige Säureamidbindungen, die im Inneren von Peptiden oder Proteinen liegen. Ihr pH-Optimum liegt meist im deutlich alkalischen Bereich. Einen Überblick über diese Familie bietet z.B. der Artikel "Subtilases: Subtilisin-like Proteases" von R. Siezen, Seite 75-95 in "Subtilisin enzymes", herausgegeben von R. Bott und C. Betzel, New York, 1996. Subtilasen werden natürlicherweise von Mikroorganismen gebildet. Hierunter sind insbesondere die von *Bacillus*-Spezies gebildeten und sezernierten Subtilisine als bedeutendste Gruppe innerhalb der Subtilasen zu erwähnen.

Beispiele für die in Wasch- und Reinigungsmitteln bevorzugt eingesetzten Proteasen vom Subtilisin-Typ sind die Subtilisine BPN' und Carlsberg, die Protease PB92, die Subtilisine 147 und 309, die Protease aus *Bacillus lentus,* insbesondere aus *Bacillus lentus* DSM 5483, Subtilisin DY und die den Subtilasen, nicht mehr jedoch den Subtilisinen im engeren Sinne zuzuordnenden Enzyme Thermitase, Proteinase K und die Proteasen TW3 und TW7, sowie Varianten der genannten Proteasen, die eine gegenüber der Ausgangsprotease veränderte Aminosäuresequenz aufweisen. Proteasen werden durch aus dem Stand der Technik bekannte Verfahren gezielt oder zufallsbasiert verändert und so z.B. für den Einsatz in Wasch- und Reinigungsmitteln optimiert. Dazu gehören Punktmutagenese, Deletions- oder Insertionsmutagenese oder Fusion mit anderen Proteinen oder Proteinteilen. So sind für die meisten aus dem Stand der Technik bekannten Proteasen entsprechend optimierte Varianten bekannt.

Generell sind nur ausgewählte Proteasen für den Einsatz in flüssigen Tensid-haltigen Zubereitungen überhaupt geeignet. Viele Proteasen zeigen in derartigen Zubereitungen keine ausreichende katalytische Leistung. Für die Anwendung von Proteasen in Reinigungsmitteln ist daher eine hohe katalytische Aktivität unter Bedingungen wie sie sich während eines Waschvorgangs darstellen besonders wünschenswert.

Überraschenderweise wurde jetzt festgestellt, dass eine Protease aus *Bacillus gibsonii* oder eine hierzu hinreichend ähnliche Protease (bezogen auf die Sequenzidentität), die eine Aminosäuresubstitution an mindestens einer der Positionen, die den Positionen 12, 43, 122, 127, 154, 156, 160, 211, 212 und 222, jeweils bezogen auf die Nummerierung gemäß SEQ ID NO:1, entsprechen, aufweist, hinsichtlich der proteolytischen Aktivität unter Standard-Waschbedingungen gegenüber der Wildtypform verbessert ist und daher besonders für den Einsatz in Wasch- oder Reinigungsmitteln geeignet ist.

Gegenstand der Erfindung ist daher in einem ersten Aspekt eine Protease umfassend eine Aminosäuresequenz, die mindestens 70% Sequenzidentität mit der in SEQ ID NO:1 angegebenen Aminosäuresequenz über deren Gesamtlänge aufweist und eine Aminosäuresubstitution an mindestens einer der Positionen, die den Positionen 12, 43, 122, 127, 154, 156, 160, 211, 212 und 222, jeweils bezogen auf die Nummerierung gemäß SEQ ID NO:1, entsprechen, aufweist.

Ein weiterer Gegenstand der Erfindung ist ein Verfahren zur Herstellung einer Protease umfassend das Substituieren einer Aminosäure an mindestens einer Position, die der Position Positionen 12, 43, 122, 127, 154, 156, 160, 211, 212 oder 222 in SEQ ID NO:1 entspricht, in einer Ausgangsprotease, die mindestens 70% Sequenzidentität zu der in SEQ ID NO:1 angegebenen Aminosäuresequenz über deren Gesamtlänge aufweist, vorzugsweise derart, dass die Protease mindestens eine der Aminosäuresubstitutionen Q12L, I43V, M122L, D127P, N154S, T156A, G160S, M211N, M211L, P212D, P212H oder A222S aufweist.

Eine Protease im Sinne der vorliegenden Patentanmeldung umfasst daher sowohl die Protease als solche als auch eine mit einem erfindungsgemäßen Verfahren hergestellte Protease. Alle Ausführungen zur Protease beziehen sich daher sowohl auf die Protease als solche wie auch auf die mittels entsprechender Verfahren hergestellten Proteasen.

Weitere Aspekte der Erfindung betreffen die für diese Proteasen codierenden Nukleinsäuren, erfindungsgemäße Proteasen oder Nukleinsäuren enthaltende nicht menschliche Wirtszellen sowie erfindungsgemäße Proteasen umfassende Mittel, insbesondere Wasch- und Reinigungsmittel, Wasch- und Reinigungsverfahren, und Verwendungen der erfindungsgemäßen Proteasen in Wasch- oder Reinigungsmitteln zur Entfernung von proteinhaltigen Anschmutzungen.

"Mindestens eine", wie hierin verwendet, bedeutete eine oder mehrere, d.h. 1, 2, 3, 4, 5, 6, 7, 8, 9, 10, 11, 12, 13, 14 oder mehr.

Die vorliegende Erfindung basiert auf der überraschenden Erkenntnis der Erfinder, dass eine Aminosäuresubstitution an mindestens einer der Positionen, die den Positionen 12, 43, 122, 127, 154, 156, 160, 211, 212 oder 222 der Protease aus *Bacillus gibsonii* gemäß SEQ ID NO:1 entsprechen, in einer Protease, die eine zu der in SEQ ID NO:1 angegebenen Aminosäuresequenz zu mindestens 70% identische Aminosäuresequenz umfasst, derart, dass an mindestens einer der entsprechenden Positionen die Aminosäuren 12L, 43V, 122L, 127P, 154S, 156A, 160S, 211N, 211L, 212D, 212H oder 222S vorhanden sind, eine verbesserte katalytische Aktivität dieser veränderten Protease in Wasch- und Reinigungsmitteln bewirkt. Das ist insbesondere insoweit überraschend, als dass keine der oben genannten Aminosäuresubstitutionen zuvor mit einer erhöhten katalytischen Aktivität der Protease in Verbindung gebracht wurde. In verschiedenen Ausführungsformen sind die Ausgangs-Aminosäuren an den vorstehend genannten Positionen, d.h. die zu substituierenden Aminosäuren, Q12, I43, M122, D127, N154, T156, G160, M211, M211, P212, P212 und/oder A222.

Die erfindungsgemäßen Proteasen verfügen über eine erhöhte katalytische Aktivität in Wasch- oder Reinigungsmitteln. In vielfältigen Ausführungsformen besitzen die erfindungsgemäßen Proteasen eine proteolytische Aktivität, die bezogen auf die Wildtyp-Variante der Protease (SEQ ID NO:1) mindestens 110%, mindestens 115%, mindestens 120%, mindestens 125%, mindestens 130%, mindestens 135%, mindestens 140%, mindestens 145%, mindestens 150%, mindestens 155% oder mindestens 160% beträgt. Solche leistungsverbesserten Proteasen ermöglichen verbesserte Waschergebnisse an proteolytisch-sensitiven Anschmutzungen in verschiedenen Temperaturbereichen, insbesondere einem Temperaturbereich von 40°C bis 60°C.

Die erfindungsgemäßen Proteasen weisen enzymatische Aktivität auf, d.h., sie sind zur Hydrolyse von Peptiden und Proteinen befähigt, insbesondere in einem Wasch- oder Reinigungsmittel. Eine erfindungsgemäße Protease ist daher ein Enzym, welches die Hydrolyse von Amid/Peptidbindungen in Protein/Peptid-Substraten katalysiert und dadurch in der Lage ist, Proteine oder Peptide zu spalten. Ferner handelt es sich bei einer erfindungsgemäßen Protease vorzugsweise um eine reife (mature) Protease, d.h. um das katalytisch aktive Molekül ohne Signal- und/oder Propeptid(e). Soweit nicht anders angegeben beziehen sich auch die angegebenen Sequenzen auf jeweils reife (prozessierte) Enzyme.

In verschiedenen Ausführungsformen der Erfindung ist die Protease ein frei vorliegendes Enzym. Dies bedeutet, dass die Protease mit allen Komponenten eines Mittels direkt agieren kann und, falls es sich bei dem Mittel um ein Flüssigmittel handelt, dass die Protease direkt mit dem Lösungsmittel des Mittels (z.B. Wasser) in Kontakt steht. In anderen Ausführungsformen kann ein Mittel Proteasen enthalten, die einen Interaktionskomplex mit anderen Molekülen bilden oder die eine "Umhüllung" enthalten. Hierbei kann ein einzelnes oder mehrere Protease Moleküle durch eine sie umgebende Struktur von den anderen Bestandteilen des Mittels getrennt sein. Eine solche trennende Struktur kann entstehen durch, ist allerdings nicht beschränkt auf, Vesikel, wie etwa eine Micelle oder ein Liposom. Die umgebende Struktur kann aber auch ein Viruspartikel, eine bakterielle Zelle oder eine eukaryotische Zelle sein. In verschiedenen Ausführungsformen kann ein Mittel Zellen von *Bacillus gibsonii* oder *Bacillus subtilus,* die die erfindungsgemäßen Proteasen exprimieren, oder Zellkulturüberstände solcher Zellen enthalten.

Ferner enthält die erfindungsgemäße Protease in verschiedenen Ausführungsformen mindestens eine Aminosäuresubstitution, die aus der Gruppe bestehend aus Q12L, I43V, M122L, D127P, N154S, T156A, G160S, M211N, M211L, P212D, P212H oder A222S, jeweils bezogen auf die Nummerierung gemäß SEQ ID NO:1, ausgewählt ist. In einer weiter bevorzugten Ausführungsformen enthält die erfindungsgemäße Protease eine der folgenden Aminosäuresubstitutionsvarianten: (I) I43V; (II) M122L, N154S und T156A; (III) M211N und P212D; (IV) M211L und P212D; (V) G160S; (VI) D127P, M211L und P212D; (VII) P212H; oder (VIII) Q12L, M122L und A222S, wobei die Nummerierung jeweils bezogen ist auf die Nummerierung gemäß SEQ ID NO:1.

In einer weiteren Ausführungsform der Erfindung umfasst die Protease eine Aminosäuresequenz, die zu der in SEQ ID NO:1 angegebenen Aminosäuresequenz über deren Gesamtlänge zu mindestens 70%, 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist, und die an mindestens einer der Positionen, die den Positionen 12, 43, 122, 127, 154, 156, 160, 211, 212 oder 222 in der Zählung gemäß SEQ ID NO:1 entsprechen, eine oder mehrere der Aminosäuresubstitutionen 12L, 43V, 122L, 127P, 154S, 156A, 160S, 211N, 211L, 212D, 212H oder 222S aufweist. Im Zusammenhang mit der vorliegenden Erfindung bedeutet das Merkmal, dass eine Protease die angegebenen Substitutionen aufweist, dass sie mindestens eine der entsprechenden Aminosäuren an den entsprechenden Positionen enthält, d.h. nicht alle der 10 Positionen anderweitig mutiert oder, z.B. durch Fragmentierung der Protease, deletiert sind. Die Aminosäuresequenzen derartiger Proteasen, die erfindungsgemäß bevorzugt sind, sind in SEQ ID Nos: 2-9 angegeben.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. z.B. Altschul et al. (1990) Basic local alignment search tool, J. Mol. Biol. 215, 403-410, und Altschul et al. (1997) Gapped BLAST and PSI-BLAST: a new generation of protein database search programs, Nucleic Acids Res., 25, 3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. z.B. Chenna et al. (2003) Multiple sequence alignment with the Clustal series of programs, Nucleic Acid Res., 31, 3497-3500), T-Coffee (vgl. z.B. Notredame et al. (2000) T-Coffee: A novel method for multiple sequence alignments, J. Mol. Biol. 302, 205-217) oder Programme, die auf diesen Programmen bzw. Algorithmen basieren. Ferner möglich sind Sequenzvergleiche (Alignments) mit dem Computer-Programm Vector NTI^{®} Suite 10.3 (Invitrogen Corporation, 1600 Faraday Avenue, Carlsbad, Kalifornien, USA) mit den vorgegebenen Standardparametern, dessen AlignX-Modul für die Sequenzvergleiche auf ClustalW basiert. Soweit nicht anders angegeben, wird die hierin angegebene Sequenzidentität mit dem BLAST-Algorithmus bestimmt.

Solch ein Vergleich erlaubt auch eine Aussage über die Ähnlichkeit der verglichenen Sequenzen zueinander. Sie wird üblicherweise in Prozent Identität, d.h. dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essenzielle Funktionen aus. Es kann daher sinnvoll sein, Sequenzübereinstimmungen nur auf einzelne, ggf. kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Im Zusammenhang mit der vorliegenden Erfindung bedeutet die Angabe, dass eine Aminosäureposition einer numerisch bezeichneten Position in SEQ ID NO:1 entspricht daher, dass die entsprechende Position der numerisch bezeichneten Position in SEQ ID NO:1 in einem wie oben definierten Alignment zugeordnet ist.

In einer weiteren Ausführungsform der Erfindung ist die Protease dadurch gekennzeichnet, dass ihre Reinigungsleistung gegenüber derjenigen einer Protease, die eine Aminosäuresequenz umfasst, die der in SEQ ID NO:1 angegebenen Aminosäuresequenz entspricht, nicht signifikant verringert ist, d.h. mindestens 80% der Referenzwaschleistung besitzt, vorzugsweise mindestens 100%, noch bevorzugter mindestens 110% oder mehr. Die Reinigungsleistung kann in einem Waschsystem bestimmt werden, das ein automatisches Geschirrspülmittel in einer Dosierung in wie hierin angegeben sowie die Protease enthält, wobei die zu vergleichenden Proteasen konzentrationsgleich (bezogen auf aktives Protein) eingesetzt sind und die Reinigungsleistung gegenüber einer Anschmutzung von Tee, Fleisch, Spaghetti und/oder Creme Brûlée bestimmt wird durch Messung des Reinigungsgrades des gewaschenen Geschirrs. Beispielsweise kann der Waschvorgang für 57 Minuten bei einer Temperatur von 44°C erfolgen und das Wasser eine Wasserhärte zwischen 5 und 25°, bevorzugt 10 und 22°, bevorzugter 18 und 22° und ferner bevorzugt 20,5 und 21,5° (deutsche Härte) aufweisen. Die Konzentration der Protease in dem für dieses Waschsystem bestimmten Reinigungsmittels beträgt 0,001-0,1 Gew.-%, vorzugsweise 0,01 bis 0,06 Gew.-%, bezogen auf aktives, gereinigtes Protein.

Ein flüssiges Referenzmittel für ein solches Waschsystem kann wie in Tabelle 2 angegeben zusammengesetzt sein.

Im Rahmen der Erfindung erfolgt die Bestimmung der Reinigungsleistung z.B. bei 45°C unter Verwendung eines Geschirrspülmittels wie vorstehend angegeben, wobei der Waschvorgang vorzugsweise für 57 Minuten mit 8 Minuten Haltezeit erfolgt.

Durch den aktivitätsgleichen Einsatz der jeweiligen Protease wird sichergestellt, dass auch bei einem etwaigen Auseinanderklaffen des Verhältnisses von Aktivsubstanz zu Gesamtprotein (die Werte der spezifischen Aktivität) die jeweiligen enzymatischen Eigenschaften, also z.B. die Reinigungsleistung an bestimmten Anschmutzungen, verglichen werden. Generell gilt, dass eine niedrige spezifische Aktivität durch Zugabe einer größeren Proteinmenge ausgeglichen werden kann. Ferner können die zu untersuchenden Enzyme auch in gleicher Stoffmenge oder Gewichtsmenge eingesetzt werden, falls die zu untersuchenden Enzyme in einem Aktivitätstest eine unterschiedliche Affinität an das Testsubstrat aufweisen. Der Ausdruck "gleiche Stoffmenge" bezieht sich in diesem Zusammenhang auf eine molgleiche Verwendung der zu untersuchenden Enzyme. Der Ausdruck "gleiche Gewichtsmenge" bezieht sich auf einen gewichtsgleichen Einsatz der zu untersuchenden Enzyme.

Ansonsten sind Verfahren zur Bestimmung der Proteaseaktivität dem Fachmann auf dem Gebiet der Enzymtechnologie geläufig und werden von ihm routinemäßig angewendet. Beispielsweise sind solche Verfahren offenbart in Tenside, Band 7 (1970), S. 125-132. Alternativ kann die Protease-Aktivität über die Freisetzung des Chromophors para-Nitroanilin (pNA) aus dem Substrat suc-L-Ala-L-Ala-L-Pro-L-Phe-p-Nitroanilid (AAPF) bestimmt werden. Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979). Die Messung erfolgt bei einer Temperatur von 25°C, bei pH 8,6, und einer Wellenlänge von 410 nm. Die Messzeit beträgt 5 min und das Messintervall 20s bis 60s. Die Proteaseaktivität wird üblicherweise in Protease-Einheiten (PE) angegeben. Geeignete Proteaseaktivitäten betragen z.B. 2,25, 5 oder 10 PE pro ml Spülflotte oder Spülvorgang. Die Proteaseaktivität ist jedoch nicht gleich Null.

Ein alternativer Test zur Feststellung der proteolytischen Aktivität der erfindungsgemäßen Proteasen ist ein optisches Messverfahren, bevorzugt ein photometrisches Verfahren. Der hierfür geeignete Test umfasst die Protease-abhängige Spaltung des Substratproteins Casein. Dieses wird durch die Protease in eine Vielzahl kleinerer Teilprodukte gespalten. Die Gesamtheit dieser Teilprodukte weist eine erhöhte Absorption bei 290 nm gegenüber nicht gespaltenem Casein auf, wobei diese erhöhte Absorption unter Verwendung eines Photometers ermittelt werden und somit ein Rückschluss auf die enzymatische Aktivität der Protease gezogen werden kann.

Die Proteinkonzentration kann mit Hilfe bekannter Methoden, z.B. dem BCA-Verfahren (Bicinchoninsäure; 2,2'-Bichinolyl-4,4'-dicarbonsäure) oder dem Biuret-Verfahren (Gornall et al. (1948), J. Biol. Chem., 177, 751-766) bestimmt werden. Die Bestimmung der Aktivproteinkonzentration kann diesbezüglich über eine Titration der aktiven Zentren unter Verwendung eines geeigneten irreversiblen Inhibitors und Bestimmung der Restaktivität (vgl. Bender et al. (1966), J. Am. Chem. Soc. 88, 24, 5890-5913) erfolgen.

Zusätzlich zu den vorstehend erläuterten Aminosäureveränderungen können erfindungsgemäße Proteasen weitere Aminosäureveränderungen, insbesondere AminosäureSubstitutionen, -Insertionen oder -Deletionen, aufweisen. Solche Proteasen sind z.B. durch gezielte genetische Veränderung, d.h. durch Mutageneseverfahren, weiterentwickelt und für bestimmte Einsatzzwecke oder hinsichtlich spezieller Eigenschaften (z.B. hinsichtlich ihrer katalytischen Aktivität, Stabilität, usw.) optimiert. Ferner können erfindungsgemäße Nukleinsäuren in Rekombinationsansätze eingebracht und damit zur Erzeugung völlig neuartiger Proteasen oder anderer Polypeptide genutzt werden.

Das Ziel ist es, in die bekannten Moleküle gezielte Mutationen wie Substitutionen, Insertionen oder Deletionen einzuführen, um z.B. die Reinigungsleistung von erfindungsgemäßen Enzymen zu verbessern. Hierzu können insbesondere die Oberflächenladungen und/oder der isoelektrische Punkt der Moleküle und dadurch ihre Wechselwirkungen mit dem Substrat verändert werden. So kann z.B. die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität oder katalytische Aktivität der Protease erhöht und dadurch ihre Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierter Proteasen, z.B. hinsichtlich ihrer Stabilität bei Lagerung, kann daher im Rahmen der Erfindung zusätzlich weiterentwickelt sein.

Für die Beschreibung von Substitutionen, die genau eine Aminosäureposition betreffen (Aminosäureaustausche), wird hierin folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere Austausche innerhalb derselben Polypeptidkette werden durch Schrägstriche voneinander getrennt. Bei Insertionen sind nach der Sequenzposition zusätzliche Aminosäuren benannt. Bei Deletionen ist die fehlende Aminosäure durch ein Symbol, z.B. einen Stern oder einen Strich, ersetzt oder vor der entsprechenden Position ein Δ angegeben. Beispielsweise beschreibt P14H die Substitution von Prolin an Position 14 durch Histidin, P14HT die Insertion von Threonin nach der Aminosäure Histidin an Position 14 und P14* oder ΔP14 die Deletion von Prolin an Position 14. Diese Nomenklatur ist dem Fachmann auf dem Gebiet der Enzymtechnologie bekannt.

Ein weiterer Gegenstand der Erfindung ist daher eine Protease, die dadurch gekennzeichnet ist, dass sie aus einer Protease wie vorstehend beschrieben als Ausgangsmolekül erhältlich ist durch ein- oder mehrfache konservative Aminosäuresubstitution, wobei die Protease in der Zählung gemäß SEQ ID NO:1 noch mindestens eine der erfindungsgemäßen Aminosäuresubstitutionen an den Positionen, die Positionen 12, 43, 122, 127, 154, 156, 160, 211, 212 und 222 in SEQ ID NO:1 entsprechen, aufweist, wie vorstehend beschrieben. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z. B. der Austausch eines unpolaren Aminosäurerestes gegen einen anderen unpolaren Aminosäurerest. Konservative Aminosäuresubstitutionen im Rahmen der Erfindung umfassen beispielsweise: G=A=S, I=V=L=M, D=E, N=Q, K=R, Y=F, S=T, G=A=I=V=L=M=Y=F=W=P=S=T.

Alternativ oder ergänzend ist die Protease dadurch gekennzeichnet, dass sie aus einer erfindungsgemäßen Protease als Ausgangsmolekül erhältlich ist durch Fragmentierung, Deletions-, Insertions- oder Substitutionsmutagenese und eine Aminosäuresequenz umfasst, die über eine Länge von mindestens 190, 200, 210, 220, 230, 240, 250, 260 oder 269 zusammenhängenden Aminosäuren mit dem Ausgangsmolekül übereinstimmt, wobei die im Ausgangsmolekül enthaltene(n) Aminosäuresubstitution(en) an einer oder mehreren der Positionen, die Positionen 12, 43, 122, 127, 154, 156, 160, 211, 212 und 222 in SEQ ID NO:1 entsprechen, noch vorhanden ist/sind.

So ist es z.B. möglich, an den Termini oder in den Loops des Enzyms einzelne Aminosäuren zu deletieren, ohne dass dadurch die proteolytische Aktivität verloren oder vermindert wird. Ferner kann durch derartige Fragmentierung, Deletions-, Insertions- oder Substitutionsmutagenese z.B. auch die Allergenizität betreffender Enzyme gesenkt und somit insgesamt ihre Einsetzbarkeit verbessert werden. Vorteilhafterweise behalten die Enzyme auch nach der Mutagenese ihre proteolytische Aktivität, d.h. ihre proteolytische Aktivität entspricht mindestens derjenigen des Ausgangsenzyms, d.h. in einer bevorzugten Ausführungsform beträgt die proteolytische Aktivität mindestens 80%, vorzugsweise mindestens 90% der Aktivität des Ausgangsenzyms. Auch weitere Substitutionen können vorteilhafte Wirkungen zeigen. Sowohl einzelne wie auch mehrere zusammenhängende Aminosäuren können gegen andere Aminosäuren ausgetauscht werden.

Alternativ oder ergänzend ist die Protease dadurch gekennzeichnet, dass sie aus einer erfindungsgemäßen Protease als Ausgangsmolekül erhältlich ist durch ein- oder mehrfache konservative Aminosäuresubstitution, wobei die Protease mindestens eine der Aminosäuresubstitutionen Q12L, I43V, M122L, D127P, N154S, T156A, G160S, M211N, M211L, P212D, P212H oder A222S an den Positionen, die den Positionen 12, 43, 122, 127, 154, 156, 160, 211, 212 und 222 gemäß SEQ ID NO:1 entsprechen, aufweist.

In weiteren Ausführungsformen ist die Protease dadurch gekennzeichnet, dass sie aus einer erfindungsgemäßen Protease als Ausgangsmolekül erhältlich ist durch Fragmentierung, Deletions-, Insertions- oder Substitutionsmutagenese und eine Aminosäuresequenz umfasst, die über eine Länge von mindestens 190, 200, 210, 220, 230, 240, 250, 260 oder 269 zusammenhängenden Aminosäuren mit dem Ausgangsmolekül übereinstimmt, wobei die Protease mindestens eine der Aminosäuresubstitutionen Q12L, I43V, M122L, D127P, N154S, T156A, G160S, M211N, M211L, P212D, P212H oder A222S an den Positionen, die den Positionen 12, 43, 122, 127, 154, 156, 160, 211, 212 und 222 gemäß SEQ ID NO:1 entsprechen, umfasst.

Die weiteren Aminosäurepositionen werden hierbei durch ein Alignment der Aminosäuresequenz einer erfindungsgemäßen Protease mit der Aminosäuresequenz der Protease aus *Bacillus gibsonii,* wie sie in SEQ ID NO:1 angegeben ist, definiert. Weiterhin richtet sich die Zuordnung der Positionen nach dem reifen (maturen) Protein. Diese Zuordnung ist insbesondere auch anzuwenden, wenn die Aminosäuresequenz einer erfindungsgemäßen Protease eine höhere Zahl von Aminosäureresten umfasst als die Protease aus *Bacillus gibsonii* gemäß SEQ ID NO:1. Ausgehend von den genannten Positionen in der Aminosäuresequenz der Protease aus *Bacillus gibsonii* sind die Veränderungspositionen in einer erfindungsgemäßen Protease diejenigen, die eben diesen Positionen in einem Alignment zugeordnet sind.

Vorteilhafte Positionen für Sequenzveränderungen, insbesondere Substitutionen, der Protease aus *Bacillus gibsonii,* die übertragen auf homologe Positionen der erfindungsgemäßen Proteasen bevorzugt von Bedeutung sind und der Protease vorteilhafte funktionelle Eigenschaften verleihen, sind demnach die Positionen, die in einem Alignment den Positionen 12, 43, 122, 127, 154, 156, 160, 211, 212 und 222 in SEQ ID NO:1 entsprechen, d.h. in der Zählung gemäß SEQ ID NO:1. An den genannten Positionen liegen in dem Wildtypmolekül der Protease aus *Bacillus gibsonii* folgende Aminosäurereste: Q12, I43, M122, D127, N154, T156, G160, M211, P212 und A222.

Eine weitere Bestätigung der korrekten Zuordnung der zu verändernden Aminosäuren, d.h. insbesondere deren funktionelle Entsprechung, können Vergleichsversuche liefern, wonach die beiden auf der Basis eines Alignments einander zugeordneten Positionen in beiden miteinander verglichenen Proteasen auf die gleiche Weise verändert werden und beobachtet wird, ob bei beiden die enzymatische Aktivität auf gleiche Weise verändert wird. Geht z.B. ein Aminosäureaustausch in einer bestimmten Position der Protease aus *Bacillus gibsonii* gemäß SEQ ID NO:1 mit einer Veränderung eines enzymatischen Parameters einher, z.B. mit der Erhöhung des K_{M}-Wertes, und wird eine entsprechende Veränderung des enzymatischen Parameters, z.B. also ebenfalls eine Erhöhung des K_{M}-Wertes, in einer erfindungsgemäßen Protease-Variante beobachtet, deren Aminosäureaustausch durch dieselbe eingeführte Aminosäure erreicht wurde, so ist hierin eine Bestätigung der korrekten Zuordnung zu sehen.

Alle genannten Sachverhalte sind auch auf die erfindungsgemäßen Verfahren zur Herstellung einer Protease anwendbar. Demnach umfasst ein erfindungsgemäßes Verfahren ferner einen oder mehrere der folgenden Verfahrensschritte:
a) Einbringen einer ein- oder mehrfachen konservativen Aminosäuresubstitution, wobei die Protease mindestens eine der Aminosäuresubstitutionen Q12L, I43V, M122L, D127P, N154S, T156A, G160S, M211N, M211L, P212D, P212H oder A222S an den Positionen, die den Positionen 12, 43, 122, 127, 154, 156, 160, 211, 212 und 222 gemäß SEQ ID NO:1 entsprechen, umfasst;
b) Veränderung der Aminosäuresequenz durch Fragmentierung, Deletions-, Insertions- oder Substitutionsmutagenese derart, dass die Protease eine Aminosäuresequenz umfasst, die über eine Länge von mindestens 190, 200, 210, 220, 230, 240, 250, 260 oder 269 zusammenhängenden Aminosäuren mit dem Ausgangsmolekül übereinstimmt, wobei die Protease mindestens eine der Aminosäuresubstitutionen Q12L, I43V, M122L, D127P, N154S, T156A, G160S, M211N, M211L, P212D, P212H oder A222S an den Positionen, die den Positionen 12, 43, 122, 127, 154, 156, 160, 211, 212 und 222 gemäß SEQ ID NO:1 entsprechen, umfasst.

Sämtliche Ausführungen gelten auch für die erfindungsgemäßen Verfahren.

In weiteren Ausgestaltungen der Erfindung ist die Protease bzw. die mit einem erfindungsgemäßen Verfahren hergestellte Protease noch mindestens zu 70%, 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, oder 98,8% identisch zu der in SEQ ID NO:1 angegebenen Aminosäuresequenz über deren Gesamtlänge. Alternativ ist die Protease bzw. die mit einem erfindungsgemäßen Verfahren hergestellte Protease noch mindestens zu 70%, 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, oder 98% identisch zu einer der in SEQ ID Nos:2-9 angegebenen Aminosäuresequenzen über deren Gesamtlänge. Die Protease bzw. die mit einem erfindungsgemäßen Verfahren hergestellte Protease weist eine Aminosäuresubstitution an mindestens einer der Positionen, die den Positionen Q12, I43, M122, D127, N154, T156, G160, M211, P212 oder A222, jeweils bezogen auf die Nummerierung gemäß SEQ ID NO:1, entsprechen, auf. In bevorzugteren Ausführungsformen ist die Aminosäuresubstitution mindestens eine ausgewählt aus der Gruppe bestehend aus Q12L, I43V, M122L, D127P, N154S, T156A, G160S, M211N, M211L, P212D, P212H und A222S, jeweils bezogen auf die Nummerierung gemäß SEQ ID NO:1. In weiter bevorzugten Ausführungsformen umfasst die Protease eine der folgenden Aminosäuresubstitutionsvarianten: (I) I43V; (II) M122L, N154S und T156A; (III) M211N und P212D; (IV) M211L und P212D; (V) G160S; (VI) D127P, M211L und P212D; (VII) P212H; oder (VIII) Q12L, M122L und A222S, wobei die Nummerierung jeweils bezogen ist auf die Nummerierung gemäß SEQ ID NO:1.

Ein weiterer Gegenstand der Erfindung ist eine zuvor beschriebene Protease, die zusätzlich stabilisiert ist, insbesondere durch eine oder mehrere Mutationen, z.B. Substitutionen, oder durch Kopplung an ein Polymer. Eine Erhöhung der Stabilität bei der Lagerung und/oder während des Einsatzes, z.B. beim Waschprozess, führt dazu, dass die enzymatische Aktivität länger anhält und damit die Reinigungsleistung verbessert wird. Grundsätzlich kommen alle im Stand der Technik beschriebenen und/oder zweckmäßigen Stabilisierungsmöglichkeiten in Betracht. Bevorzugt sind solche Stabilisierungen, die über Mutationen des Enzyms selbst erreicht werden, da solche Stabilisierungen im Anschluss an die Gewinnung des Enzyms keine weiteren Arbeitsschritte erfordern. Beispiele für hierfür geeignete Sequenzveränderungen sind vorstehend genannt. Weitere geeignete Sequenzveränderungen sind aus dem Stand der Technik bekannt.

Weitere Möglichkeiten der Stabilisierung sind z.B.:
- Veränderung der Bindung von Metallionen, insbesondere der Calcium-Bindungsstellen, z.B. durch Austauschen von einer oder mehreren der an der Calcium-Bindung beteiligten Aminosäure(n) gegen eine oder mehrere negativ geladene Aminosäuren und/oder durch Einführen von Sequenzveränderungen in mindestens einer der Folgen der beiden Aminosäuren Arginin/Glycin;
- Schutz gegen den Einfluss von denaturierenden Agentien wie Tensiden durch Mutationen, die eine Veränderung der Aminosäuresequenz auf oder an der Oberfläche des Proteins bewirken;
- Austausch von Aminosäuren, die nahe dem N-Terminus liegen, gegen solche, die vermutlich über nicht-kovalente Wechselwirkungen mit dem Rest des Moleküls in Kontakt treten und somit einen Beitrag zur Aufrechterhaltung der globulären Struktur leisten.

Bevorzugte Ausführungsformen sind solche, bei denen das Enzym auf mehrere Arten stabilisiert wird, da mehrere stabilisierende Mutationen additiv oder synergistisch wirken.

Ein weiterer Gegenstand der Erfindung ist eine Protease wie vorstehend beschrieben, die dadurch gekennzeichnet ist, dass sie mindestens eine chemische Modifikation aufweist. Eine Protease mit einer solchen Veränderung wird als Derivat bezeichnet, d.h. die Protease ist derivatisiert.

Unter Derivaten werden im Sinne der vorliegenden Anmeldung demnach solche Proteine verstanden, deren reine Aminosäurekette chemisch modifiziert worden ist. Solche Derivatisierungen können z.B. *in vivo* durch die Wirtszelle erfolgen, die das Protein exprimiert. Diesbezüglich sind Kopplungen niedrigmolekularer Verbindungen wie von Lipiden oder Oligosacchariden besonders hervorzuheben. Derivatisierungen können aber auch *in vitro* durchgeführt werden, etwa durch die chemische Umwandlung einer Seitenkette einer Aminosäure oder durch kovalente Bindung einer anderen Verbindung an das Protein. Beispielsweise ist die Kopplung von Aminen an Carboxylgruppen eines Enzyms zur Veränderung des isoelektrischen Punkts möglich. Eine solche andere Verbindung kann auch ein weiteres Protein sein, das z.B. über bifunktionelle chemische Verbindungen an ein erfindungsgemäßes Protein gebunden wird. Ebenso ist unter Derivatisierung die kovalente Bindung an einen makromolekularen Träger zu verstehen, oder auch ein nichtkovalenter Einschluss in geeignete makromolekulare Käfigstrukturen. Derivatisierungen können z.B. die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann z.B. für den Zeitraum der Lagerung sinnvoll sein. Derartige Modifikationen können ferner die Stabilität oder die enzymatische Aktivität beeinflussen. Sie können ferner auch dazu dienen, die Allergenizität und/oder Immunogenizität des Proteins herabzusetzen und damit z.B. dessen Hautverträglichkeit zu erhöhen. Beispielsweise können Kopplungen mit makromolekularen Verbindungen, z.B. Polyethylenglykol, das Protein hinsichtlich der Stabilität und/oder Hautverträglichkeit verbessern.

Unter Derivaten eines erfindungsgemäßen Proteins können im weitesten Sinne auch Präparationen dieser Proteine verstanden werden. Je nach Gewinnung, Aufarbeitung oder Präparation kann ein Protein mit diversen anderen Stoffen kombiniert sein, z.B. aus der Kultur der produzierenden Mikroorganismen. Ein Protein kann auch, z.B. zur Erhöhung seiner Lagerstabilität, mit anderen Stoffen gezielt versetzt worden sein. Erfindungsgemäß sind deshalb auch alle Präparationen eines erfindungsgemäßen Proteins. Das ist auch unabhängig davon, ob es in einer bestimmten Präparation tatsächlich diese enzymatische Aktivität entfaltet oder nicht. Denn es kann gewünscht sein, dass es bei der Lagerung keine oder nur geringe Aktivität besitzt, und erst zum Zeitpunkt der Verwendung seine enzymatische Funktion entfaltet. Dies kann z.B. über entsprechende Begleitstoffe gesteuert werden. Insbesondere die gemeinsame Präparation von Proteasen mit spezifischen Inhibitoren ist diesbezüglich möglich.

Unter allen vorstehend beschriebenen Proteasen bzw. Proteasevarianten und/oder Derivaten sind im Rahmen der vorliegenden Erfindung diejenigen besonders bevorzugt, deren katalytische Aktivität mindestens einer derjenigen der Proteasen gemäß SEQ ID Nos: 2-9 entspricht, und/oder deren Reinigungsleistung mindestens einer derjenigen der Proteasen gemäß SEQ ID Nos: 2-9 entspricht, wobei die Reinigungsleistung in einem Waschsystem bestimmt wird wie vorstehend beschrieben.

Ein weiterer Gegenstand der Erfindung ist eine Nukleinsäure, die für eine erfindungsgemäße Protease codiert, sowie ein Vektor enthaltend eine solche Nukleinsäure, insbesondere ein Klonierungsvektor oder ein Expressionsvektor.

Hierbei kann es sich um DNA- oder RNA-Moleküle handeln. Sie können als Einzelstrang, als ein zu diesem Einzelstrang komplementärer Einzelstrang oder als Doppelstrang vorliegen. Insbesondere bei DNA-Molekülen sind die Sequenzen beider komplementärer Stränge in jeweils allen drei möglichen Leserastern zu berücksichtigen. Ferner ist zu berücksichtigen, dass verschiedene Codons, also Basentriplets, für die gleichen Aminosäuren codieren können, so dass eine bestimmte Aminosäuresequenz von mehreren unterschiedlichen Nukleinsäuren codiert werden kann. Auf Grund dieser Degeneriertheit des genetischen Codes sind sämtliche Nukleinsäuresequenzen in diesen Erfindungsgegenstand mit eingeschlossen, die eine der vorstehend beschriebenen Proteasen codieren können. Der Fachmann ist in der Lage, diese Nukleinsäuresequenzen zweifelsfrei zu bestimmen, da trotz der Degeneriertheit des genetischen Codes einzelnen Codons definierte Aminosäuren zuzuordnen sind. Daher kann der Fachmann ausgehend von einer Aminosäuresequenz für diese Aminosäuresequenz codierende Nukleinsäuren problemlos ermitteln. Weiterhin können bei erfindungsgemäßen Nukleinsäuren ein oder mehrere Codons durch synonyme Codons ersetzt sein. Dieser Aspekt bezieht sich insbesondere auf die heterologe Expression der erfindungsgemäßen Enzyme. So besitzt jeder Organismus, z.B. eine Wirtszelle eines Produktionsstammes, eine bestimmte Codon-Verwendung. Unter Codon-Verwendung wird die Übersetzung des genetischen Codes in Aminosäuren durch den jeweiligen Organismus verstanden. Es kann zu Engpässen in der Proteinbiosynthese kommen, wenn die auf der Nukleinsäure liegenden Codons in dem Organismus einer vergleichsweise geringen Zahl von beladenen tRNA-Molekülen gegenüberstehen. Obwohl für die gleiche Aminosäure codierend führt das dazu, dass in dem Organismus ein Codon weniger effizient translatiert wird als ein synonymes Codon, das für dieselbe Aminosäure codiert. Auf Grund des Vorliegens einer höheren Anzahl von tRNA-Molekülen für das synonyme Codon kann dieses in dem Organismus effizienter translatiert werden.

Einem Fachmann ist es über heutzutage allgemein bekannte Methoden, wie z.B. die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus Sambrook, J., Fritsch, E.F. and Maniatis, T. 2001. Molecular cloning: a laboratory manual, 3. Edition Cold Spring Laboratory Press. bekannt.

Unter Vektoren werden im Sinne der vorliegenden Erfindung aus Nukleinsäuren bestehende Elemente verstanden, die als kennzeichnenden Nukleinsäurebereich eine erfindungsgemäße Nukleinsäure enthalten. Sie vermögen diese in einer Spezies oder einer Zelllinie über mehrere Generationen oder Zellteilungen hinweg als stabiles genetisches Element zu etablieren. Vektoren sind insbesondere bei der Verwendung in Bakterien spezielle Plasmide, also zirkulare genetische Elemente. Im Rahmen der vorliegenden Erfindung wird eine erfindungsgemäße Nukleinsäure in einen Vektor kloniert. Zu den Vektoren zählen z.B. solche, deren Ursprung bakterielle Plasmide, Viren oder Bacteriophagen sind, oder überwiegend synthetische Vektoren oder Plasmide mit Elementen verschiedenster Herkunft. Mit den weiteren jeweils vorhandenen genetischen Elementen vermögen Vektoren sich in den betreffenden Wirtszellen über mehrere Generationen hinweg als stabile Einheiten zu etablieren. Sie können extrachromosomal als eigene Einheiten vorliegen oder in ein Chromosom oder chromosomale DNA integrieren.

Expressionsvektoren umfassen Nukleinsäuresequenzen, die sie dazu befähigen, in den sie enthaltenden Wirtszellen, vorzugsweise Mikroorganismen, besonders bevorzugt Bakterien, zu replizieren und dort eine enthaltene Nukleinsäure zur Expression zu bringen. Die Expression wird insbesondere von dem oder den Promotoren beeinflusst, welche die Transkription regulieren. Prinzipiell kann die Expression durch den natürlichen, ursprünglich vor der zu exprimierenden Nukleinsäure lokalisierten Promotor erfolgen, aber auch durch einen auf dem Expressionsvektor bereitgestellten Promotor der Wirtszelle oder auch durch einen modifizierten oder einen völlig anderen Promotor eines anderen Organismus oder einer anderen Wirtszelle. Im vorliegenden Fall wird zumindest ein Promotor für die Expression einer erfindungsgemäßen Nukleinsäure zur Verfügung gestellt und für deren Expression genutzt. Expressionsvektoren können ferner regulierbar sein, z.B. durch Änderung der Kultivierungsbedingungen oder bei Erreichen einer bestimmten Zelldichte der sie enthaltenen Wirtszellen oder durch Zugabe von bestimmten Substanzen, insbesondere Aktivatoren der Genexpression. Ein Beispiel für eine solche Substanz ist das Galactose-Derivat Isopropyl-β-D-thiogalactopyranosid (IPTG), welches als Aktivator des bakteriellen Lactose-Operons (lac-Operons) verwendet wird. Im Gegensatz zu Expressionsvektoren wird die enthaltene Nukleinsäure in Klonierungsvektoren nicht exprimiert.

Ein weiterer Gegenstand der Erfindung ist eine nicht menschliche Wirtszelle, die eine erfindungsgemäße Nukleinsäure oder einen erfindungsgemäßen Vektor beinhaltet, oder die eine erfindungsgemäße Protease beinhaltet, insbesondere eine, die die Protease in das die Wirtszelle umgebende Medium sezerniert. Bevorzugt wird eine erfindungsgemäße Nukleinsäure oder ein erfindungsgemäßer Vektor in einen Mikroorganismus transformiert, der dann eine erfindungsgemäße Wirtszelle darstellt. Alternativ können auch einzelne Komponenten, d.h. Nukleinsäure-Teile oder -Fragmente einer erfindungsgemäßen Nukleinsäure derart in eine Wirtszelle eingebracht werden, dass die dann resultierende Wirtszelle eine erfindungsgemäße Nukleinsäure oder einen erfindungsgemäßen Vektor enthält. Dieses Vorgehen eignet sich besonders dann, wenn die Wirtszelle bereits einen oder mehrere Bestandteile einer erfindungsgemäßen Nukleinsäure oder eines erfindungsgemäßen Vektors enthält und die weiteren Bestandteile dann entsprechend ergänzt werden. Verfahren zur Transformation von Zellen sind im Stand der Technik etabliert und dem Fachmann hinlänglich bekannt. Als Wirtszellen eignen sich prinzipiell alle Zellen, das heißt prokaryotische oder eukaryotische Zellen. Bevorzugt sind solche Wirtszellen, die sich genetisch vorteilhaft handhaben lassen, was z.B. die Transformation mit der Nukleinsäure oder dem Vektor und dessen stabile Etablierung angeht, z.B. einzellige Pilze oder Bakterien. Ferner zeichnen sich bevorzugte Wirtszellen durch eine gute mikrobiologische und biotechnologische Handhabbarkeit aus. Das betrifft z.B. leichte Kultivierbarkeit, hohe Wachstumsraten, geringe Anforderungen an Fermentationsmedien und gute Produktions- und Sekretionsraten für Fremdproteine. Bevorzugte erfindungsgemäße Wirtszellen sezernieren das (transgen) exprimierte Protein in das die Wirtszellen umgebende Medium. Ferner können die Proteasen von den sie produzierenden Zellen nach deren Herstellung modifiziert werden, z.B. durch Anknüpfung von Zuckermolekülen, Formylierungen, Aminierungen, usw. Solche posttranslationale Modifikationen können die Protease funktionell beeinflussen.

Weitere bevorzugte Ausführungsformen stellen solche Wirtszellen dar, die aufgrund genetischer Regulationselemente, die z.B. auf dem Vektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine wirtschaftliche Produktion der erfindungsgemäßen Proteine. Ein Beispiel für eine solche Verbindung ist IPTG wie vorstehend beschrieben.

Bevorzugte Wirtszellen sind prokaryotische oder bakterielle Zellen. Bakterien zeichnen sich durch kurze Generationszeiten und geringe Ansprüche an die Kultivierungsbedingungen aus. Dadurch können kostengünstige Kultivierungsverfahren oder Herstellungsverfahren etabliert werden. Zudem verfügt der Fachmann bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf usw., gramnegative oder grampositive Bakterien geeignet sein.

Bei gramnegativen Bakterien wie z.B. *Escherichia coli* wird eine Vielzahl von Proteinen in den periplasmatischen Raum sezerniert, also in das Kompartiment zwischen den beiden die Zellen einschließenden Membranen. Dies kann für spezielle Anwendungen vorteilhaft sein. Ferner können auch gramnegative Bakterien so ausgestaltet werden, dass sie die exprimierten Proteine nicht nur in den periplasmatischen Raum, sondern in das das Bakterium umgebende Medium ausschleusen. Grampositive Bakterien wie z.B. Bacilli oder Actinomyceten oder andere Vertreter der *Actinomycetales* besitzen demgegenüber keine äußere Membran, so dass sezernierte Proteine sogleich in das die Bakterien umgebende Medium, in der Regel das Nährmedium, abgegeben werden, aus welchem sich die exprimierten Proteine aufreinigen lassen. Sie können aus dem Medium direkt isoliert oder weiter prozessiert werden. Zudem sind grampositive Bakterien mit den meisten Herkunftsorganismen für technisch wichtige Enzyme verwandt oder identisch und bilden meist selbst vergleichbare Enzyme, so dass sie über eine ähnliche Codon-Verwendung verfügen und ihr Protein-Syntheseapparat naturgemäß entsprechend ausgerichtet ist.

Erfindungsgemäße Wirtszellen können hinsichtlich ihrer Anforderungen an die Kulturbedingungen verändert sein, andere oder zusätzliche Selektionsmarker aufweisen oder noch andere oder zusätzliche Proteine exprimieren. Es kann sich insbesondere auch um solche Wirtszellen handeln, die mehrere Proteine oder Enzyme transgen exprimieren.

Die vorliegende Erfindung ist prinzipiell auf alle Mikroorganismen, insbesondere auf alle fermentierbaren Mikroorganismen, besonders bevorzugt auf solche der Gattung *Bacillus,* anwendbar und führt dazu, dass sich durch den Einsatz solcher Mikroorganismen erfindungsgemäße Proteine herstellen lassen. Solche Mikroorganismen stellen dann Wirtszellen im Sinne der Erfindung dar.

In einer weiteren Ausführungsform der Erfindung ist die Wirtszelle dadurch gekennzeichnet, dass sie ein Bakterium ist, bevorzugt eines, das ausgewählt ist aus der Gruppe der Gattungen von *Escherichia, Klebsiella, Bacillus, Staphylococcus, Corynebakterium, Arthrobacter, Streptomyces, Stenotrophomonas* und *Pseudomonas,* weiter bevorzugt eines, das ausgewählt ist aus der Gruppe von *Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glutamicum, Arthrobacter oxidans, Streptomyces lividans, Streptomyces coelicolor* und *Stenotrophomonas maltophilia.*

Die Wirtszelle kann aber auch eine eukaryontische Zelle sein, die dadurch gekennzeichnet ist, dass sie einen Zellkern besitzt. Einen weiteren Gegenstand der Erfindung stellt daher eine Wirtszelle dar, die dadurch gekennzeichnet ist, dass sie einen Zellkern besitzt. Im Gegensatz zu prokaryotischen Zellen sind eukaryontische Zellen in der Lage, das gebildete Protein posttranslational zu modifizieren. Beispiele dafür sind Pilze wie Actinomyceten oder Hefen wie *Saccharomyces* oder *Kluyveromyces.* Dies kann z.B. dann besonders vorteilhaft sein, wenn die Proteine im Zusammenhang mit ihrer Synthese spezifische Modifikationen erfahren sollen, die derartige Systeme ermöglichen. Zu den Modifikationen, die eukaryontische Systeme besonders im Zusammenhang mit der Proteinsynthese durchführen, gehören z.B. die Bindung niedermolekularer Verbindungen wie Membrananker oder Oligosaccharide. Derartige Oligosaccharid-Modifikationen können z.B. zur Senkung der Allergenizität eines exprimierten Proteins wünschenswert sein. Auch eine Coexpression mit den natürlicherweise von derartigen Zellen gebildeten Enzymen, wie z.B. Cellulasen, kann vorteilhaft sein. Ferner können sich z.B. thermophile pilzliche Expressionssysteme besonders zur Expression temperaturbeständiger Proteine oder Varianten eignen.

Die erfindungsgemäßen Wirtszellen werden in üblicher Weise kultiviert und fermentiert, z.B. in diskontinuierlichen oder kontinuierlichen Systemen. Im ersten Fall wird ein geeignetes Nährmedium mit den Wirtszellen beimpft und das Produkt nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen teilweise absterben aber auch nachwachsen und gleichzeitig aus dem Medium das gebildete Protein entnommen werden kann.

Erfindungsgemäße Wirtszellen werden bevorzugt verwendet, um erfindungsgemäße Proteasen herzustellen. Ein weiterer Gegenstand der Erfindung ist daher ein Verfahren zur Herstellung einer Protease umfassend
a) Kultivieren einer erfindungsgemäßen Wirtszelle, und
b) Isolieren der Protease aus dem Kulturmedium oder aus der Wirtszelle.

Dieser Erfindungsgegenstand umfasst bevorzugt Fermentationsverfahren. Fermentationsverfahren sind an sich aus dem Stand der Technik bekannt und stellen den eigentlichen großtechnischen Produktionsschritt dar, in der Regel gefolgt von einer geeigneten Aufreinigungsmethode des hergestellten Produktes, z.B. der erfindungsgemäßen Proteasen. Alle Fermentationsverfahren, die auf einem entsprechenden Verfahren zur Herstellung einer erfindungsgemäßen Protease beruhen, stellen Ausführungsformen dieses Erfindungsgegenstandes dar.

Fermentationsverfahren, die dadurch gekennzeichnet sind, dass die Fermentation über eine Zulaufstrategie durchgeführt wird, kommen insbesondere in Betracht. Hierbei werden die Medienbestandteile, die durch die fortlaufende Kultivierung verbraucht werden, zugefüttert. Hierdurch können beträchtliche Steigerungen sowohl in der Zelldichte als auch in der Zellmasse bzw. Trockenmasse und/oder insbesondere in der Aktivität der interessierenden Protease erreicht werden. Ferner kann die Fermentation auch so gestaltet werden, dass unerwünschte Stoffwechselprodukte herausgefiltert oder durch Zugabe von Puffer oder jeweils passende Gegenionen neutralisiert werden.

Die hergestellte Protease kann aus dem Fermentationsmedium geerntet werden. Ein solches Fermentationsverfahren ist gegenüber einer Isolation der Protease aus der Wirtszelle, d.h. einer Produktaufbereitung aus der Zellmasse (Trockenmasse) bevorzugt, erfordert jedoch die Zurverfügungstellung von geeigneten Wirtszellen oder von einem oder mehreren geeigneten Sekretionsmarkern oder -mechanismen und/oder Transportsystemen, damit die Wirtszellen die Protease in das Fermentationsmedium sezernieren. Ohne Sekretion kann alternativ die Isolation der Protease aus der Wirtszelle, d.h. eine Aufreinigung derselben aus der Zellmasse, erfolgen, z.B. durch Fällung mit Ammoniumsulfat oder Ethanol, oder durch chromatographische Reinigung.

Alle vorstehend ausgeführten Sachverhalte können zu Verfahren kombiniert werden, um erfindungsgemäße Protease herzustellen.

Ein weiterer Gegenstand der Erfindung ist ein Mittel, das dadurch gekennzeichnet ist, dass es eine erfindungsgemäße Protease wie vorstehend beschrieben enthält. Bevorzugt ist das Mittel als ein Wasch- oder Reinigungsmittel.

Zu diesem Erfindungsgegenstand zählen alle denkbaren Wasch- oder Reinigungsmittelarten, sowohl Konzentrate als auch unverdünnt anzuwendende Mittel, zum Einsatz im kommerziellen Maßstab, in der Waschmaschine oder bei der Handwäsche bzw. -reinigung. Dazu gehören z.B. Waschmittel für Textilien, Teppiche, oder Naturfasern, für die die Bezeichnung Waschmittel verwendet wird. Dazu gehören z.B. auch Geschirrspülmittel für Geschirrspülmaschinen (automatische Geschirrspülmittel) oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder, für die die Bezeichnung Reinigungsmittel verwendet wird, also neben manuellen und maschinellen Geschirrspülmitteln z.B. auch Scheuermittel, Glasreiniger, WC-Duftspüler, usw. Zu den Wasch- und Reinigungsmittel im Rahmen der Erfindung zählen ferner Waschhilfsmittel, die bei der manuellen oder maschinellen Textilwäsche zum eigentlichen Waschmittel hinzudosiert werden, um eine weitere Wirkung zu erzielen. Ferner zählen zu Wasch- und Reinigungsmittel im Rahmen der Erfindung auch Textilvor- und Nachbehandlungsmittel, also solche Mittel, mit denen das Wäschestück vor der eigentlichen Wäsche in Kontakt gebracht wird, z.B. zum Anlösen hartnäckiger Verschmutzungen, und auch solche Mittel, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet.

Das erfindungsgemäße Geschirrspülmittel kann ein maschinelles Geschirrspülmittel aber auch ein manuelles Geschirrspülmittel sein. Maschinelle Geschirrspülmittel sind für den Einsatz in Geschirrspülautomaten optimierte Reinigungsmittel. Vorzugsweise liegen sie in fester Form vor. Manuelle Geschirrspülmittel sind für die Handwäsche optimiert. Vorzugsweise sind manuelle Geschirrspülmittel flüssig. Die erfindungsgemäßen Mittel sind vorzugsweise maschinelle Geschirrspülmittel.

Die erfindungsgemäßen Wasch- oder Reinigungsmittel, die als pulverförmige Feststoffe, in nachverdichteter Teilchenform, als homogene Lösungen oder Suspensionen vorliegen können, können neben einer erfindungsgemäßen Protease alle bekannten und in derartigen Mitteln üblichen Inhaltsstoffe enthalten, wobei bevorzugt mindestens ein weiterer Inhaltsstoff in dem Mittel vorhanden ist. Die erfindungsgemäßen Mittel können insbesondere Tenside, Builder (Gerüststoffe), Persauerstoffverbindungen oder Bleichaktivatoren enthalten. Ferner können sie wassermischbare organische Lösungsmittel, weitere Enzyme, Sequestrierungsmittel, Elektrolyte, pH-Regulatoren und/oder weitere Hilfsstoffe wie optische Aufheller, Vergrauungsinhibitoren, Schaumregulatoren sowie Farb- und Duftstoffe sowie Kombinationen hiervon enthalten.

Insbesondere eine Kombination einer erfindungsgemäßen Protease mit einem oder mehreren weiteren Inhaltsstoff(en) des Mittels ist vorteilhaft, da ein solches Mittel in bevorzugten erfindungsgemäßen Ausgestaltungen eine verbesserte Reinigungsleistung durch sich ergebende Synergismen aufweist. Insbesondere durch die Kombination einer erfindungsgemäßen Protease mit einem Tensid und/oder einem Builder (Gerüststoff) und/oder einer Persauerstoffverbindung und/oder einem Bleichaktivator kann ein solcher Synergismus erreicht werden. Allerdings kann in bevorzugten Ausführungsformen das erfindungsgemäße Mittel keine Borsäure enthalten.

Vorteilhafte Inhaltsstoffe erfindungsgemäßer Mittel sind offenbart in der internationalen Patentanmeldung WO 2009/121725, dort beginnend auf Seite 5, vorletzter Absatz, und endend auf Seite 13 nach dem zweiten Absatz. Auf diese Offenbarung wird ausdrücklich Bezug genommen und der dortige Offenbarungsgehalt in die vorliegende Patentanmeldung einbezogen.

Ein erfindungsgemäßes Mittel enthält die Protease vorteilhafterweise in einer Menge von 2 µg bis 20 mg, vorzugsweise von 5 µg bis 17,5 mg, besonders bevorzugt von 20 µg bis 15 mg und ganz besonders bevorzugt von 50 µg bis 10 mg pro g des Mittels.

Ferner kann die in dem Mittel enthaltene Protease, und/oder weitere Inhaltsstoffe des Mittels, mit einer bei Raumtemperatur oder bei Abwesenheit von Wasser für das Enzym undurchlässigen Substanz umhüllt sein, welche unter Anwendungsbedingungen des Mittels durchlässig für das Enzym wird. Eine solche Ausführungsform der Erfindung ist somit dadurch gekennzeichnet, dass die Protease mit einer bei Raumtemperatur oder bei Abwesenheit von Wasser für die Protease undurchlässigen Substanz umhüllt ist. Weiterhin kann auch das Wasch- oder Reinigungsmittel selbst in einem Behältnis, vorzugsweise einem luftdurchlässigen Behältnis, verpackt sein, aus dem es kurz vor Gebrauch oder während des Wasch-/Spülvorgangs freigesetzt wird.

Diese Ausführungsformen der vorliegenden Erfindung umfassen alle festen, pulverförmigen, flüssigen, gelförmigen oder pastösen Darreichungsformen erfindungsgemäßer Mittel, die ggf. auch aus mehreren Phasen bestehen können sowie in komprimierter oder nicht komprimierter Form vorliegen können. Das Mittel kann als rieselfähiges Pulver vorliegen, insbesondere mit einem Schüttgewicht von 300 g/l bis 1200 g/l, insbesondere 500 g/l bis 900 g/l oder 600 g/l bis 850 g/l. Zu den festen Darreichungsformen des Mittels zählen ferner Extrudate, Granulate, Tabletten oder Pouches. Alternativ kann das Mittel auch flüssig, gelförmig oder pastös sein, z.B. in Form eines nicht-wässrigen Mittels oder einer nicht-wässrigen Paste oder in Form eines wässrigen Mittels oder einer wasserhaltigen Paste. Weiterhin kann das Mittel als Einkomponentensystem vorliegen. Solche Mittel bestehen aus einer Phase. Alternativ kann ein Mittel auch aus mehreren Phasen bestehen. Ein solches Mittel ist demnach in mehrere Komponenten aufgeteilt. Bevorzugte Darreichungsformen sind feste Formen, wie z.B. ein- oder mehrphasige Tabletten ("Tabs"), oder auch wasserarme bis wasserfreie Flüssigkeiten/Gele, beide jeweils in vorzugsweise in Einheitsdosisform.

Erfindungsgemäße Wasch- oder Reinigungsmittel können ausschließlich eine Protease enthalten. Alternativ können sie auch weitere hydrolytische Enzyme oder andere Enzyme in einer für die Wirksamkeit des Mittels zweckmäßigen Konzentration enthalten. Eine weitere Ausführungsform der Erfindung stellen somit Mittel dar, die ferner eines oder mehrere weitere Enzyme umfassen. Als weitere Enzyme bevorzugt einsetzbar sind alle Enzyme, die in dem erfindungsgemäßen Mittel eine katalytische Aktivität entfalten können, insbesondere eine Lipase, Amylase, Cellulase, Hemicellulase, Mannanase, Tannase, Xylanase, Xanthanase, Xyloglucanase, β-Glucosidase, Pektinase, Carrageenase, Perhydrolase, Oxidase, Oxidoreduktase oder andere - von den erfindungsgemäßen Protease unterscheidbare - Protease, sowie deren Gemische. Weitere Enzyme sind in dem Mittel vorteilhafterweise jeweils in einer Menge von 1 x 10⁻⁸ bis 5 Gew.-% bezogen auf aktives Protein enthalten. Zunehmend bevorzugt ist jedes weitere Enzym in einer Menge von 1 x 10⁻⁷-3 Gew.-%, von 0,00001-1 Gew.-%, von 0,00005-0,5 Gew.-%, von 0,0001 bis 0,1 Gew.-% und besonders bevorzugt von 0,0001 bis 0,05 Gew.-% in erfindungsgemäßen Mitteln enthalten, bezogen auf aktives Protein. Besonders bevorzugt zeigen die Enzyme synergistische Reinigungsleistungen gegenüber bestimmten Anschmutzungen oder Flecken, d.h. die in der Mittelzusammensetzung enthaltenen Enzyme unterstützen sich in ihrer Reinigungsleistung gegenseitig. Ganz besonders bevorzugt liegt ein solcher Synergismus vor zwischen der erfindungsgemäß enthaltenen Protease und einem weiteren Enzym eines erfindungsgemäßen Mittels, darunter insbesondere zwischen der genannten Protease und einer Amylase und/oder einer Lipase und/oder einer Mannanase und/oder einer Cellulase und/oder einer Pektinase. Synergistische Effekte können nicht nur zwischen verschiedenen Enzymen, sondern auch zwischen einem oder mehreren Enzymen und weiteren Inhaltsstoffen des erfindungsgemäßen Mittels auftreten.

In den hierin beschriebenen Reinigungsmitteln können die einzusetzenden Enzyme ferner zusammen mit Begleitstoffen, etwa aus der Fermentation, konfektioniert sein. In flüssigen Formulierungen werden die Enzyme bevorzugt als Enzymflüssigformulierung(en) eingesetzt.

Die Enzyme werden in der Regel nicht in Form des reinen Proteins, sondern vielmehr in Form stabilisierter, lager- und transportfähiger Zubereitungen bereitgestellt. Zu diesen vorkonfektionierten Zubereitungen zählen z.B. die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren oder weiteren Hilfsmitteln versetzt.

Alternativ können die Enzyme sowohl für die feste als auch für die flüssige Darreichungsform verkapselt werden, z.B. durch Sprühtrocknung oder Extrusion der Enzymlösung zusammen mit einem vorzugsweise natürlichen Polymer oder in Form von Kapseln, z.B. solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, z.B. Stabilisatoren, Emulgatoren, Pigmente, Bleich- oder Farbstoffe aufgebracht werden. Derartige Kapseln werden nach an sich bekannten Methoden, z.B. durch Schüttel- oder Rollgranulation oder in Fluid-bed-Prozessen aufgebracht. Vorteilhafterweise sind derartige Granulate, z.B. durch Aufbringen polymerer Filmbildner, staubarm und aufgrund der Beschichtung lagerstabil.

Die Enzyme können auch in wasserlösliche Filme eingebracht werden. Ein derartiger Film ermöglicht die Freisetzung der Enzyme nach Kontakt mit Wasser. Wie hier verwendet, bezieht sich "wasserlöslich" auf eine Filmstruktur, die vorzugsweise vollständig wasserlöslich ist. Jedoch sind auch Filme, die im Wesentlichen wasserlöslich sind, aber relativ kleine Mengen eines Materials in der Filmstruktur aufweisen, welches nicht wasserlöslich ist; Folien mit Materialien, die nur bei relativ hohen Wassertemperaturen oder nur unter eingeschränkten pH-Bedingungen wasserlöslich sind; und Folien, die eine relativ dünne Schicht aus wasserunlöslichem Material einschließen, alle in der Bezeichnung "wasserlöslich" miteingeschlossen. Bevorzugt besteht ein solcher Film aus (vollständig oder teilweise hydrolysiertem) Polyvinylalkohol (PVA). Der Film kann aber auch ausschließlich oder zusätzlich zum PVA enthalten Säure/Acrylat-Copolymere, vorzugsweise Methacrylsäure/Ethylacrylat-Copolymer, wie das von Belland als GBC 2580 und 2600 erhältliche; Styrol-Maleinsäureanhydrid-Copolymer (SMA) (verfügbar als Scripset (Handelsname) von Monsanto); Ethylen-Acrylsäure-Copolymer (EAA) oder durch Metallsalz neutralisiertes Ethylen-Methacrylsäure-Copolymer (EMAA), bekannt als lonomer (verfügbar von du Pont), bei welchem der Säuregehalt von EAA oder EMAA mindestens etwa 20 Mol-% beträgt; Polyether-Blockamid-Copolymer; Polyhydroxyvalerinsäure (verfügbar als Biopol (Handelsname)-Harze von Imperial Chemical Industries); Polyethylenoxid; wasserlöslichen Polyester oder Copolyester; Polyethyloxazolin (PEOX 200 von Dow); und wasserlösliches Polyurethan ein.

Weiterhin ist es möglich, zwei oder mehrere Enzyme zusammen zu konfektionieren, so dass ein einzelnes Granulat mehrere Enzymaktivitäten aufweist.

Ein weiterer Erfindungsgegenstand ist ein Verfahren zur Reinigung von Textilien oder harten Oberflächen, das dadurch gekennzeichnet ist, dass in mindestens einem Verfahrensschritt ein erfindungsgemäßes Mittel angewendet wird, oder dass in mindestens einem Verfahrensschritt eine erfindungsgemäße Protease katalytisch aktiv wird, insbesondere derart, dass die Protease in einer Menge von 40 µg bis 4 g, vorzugsweise von 50 µg bis 3 g, besonders bevorzugt von 100 µg bis 2 g und ganz besonders bevorzugt von 200 µg bis 1 g eingesetzt wird.

In verschiedenen Ausführungsformen zeichnet sich das oben beschriebene Verfahren dadurch aus, dass die Protease bei einer Temperatur von 0-100°C, bevorzugt 10-70°C, weiter bevorzugt 30-50°C und am meisten bevorzugt bei 45°C eingesetzt wird.

Hierunter fallen sowohl manuelle als auch maschinelle Verfahren, wobei maschinelle Verfahren bevorzugt sind. Verfahren zur Reinigung von Textilien zeichnen sich im Allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung oder Verdünnung dieses Mittels behandelt wird. Entsprechendes gilt für Verfahren zur Reinigung von allen anderen Materialien als Textilien, insbesondere von harten Oberflächen. Alle denkbaren Wasch- oder Reinigungsverfahren können in wenigstens einem der Verfahrensschritte um die Anwendung eines erfindungsgemäßen Wasch- oder Reinigungsmittels oder einer erfindungsgemäßen Protease bereichert werden und stellen dann Ausführungsformen der vorliegenden Erfindung dar.

Alle Sachverhalte, Gegenstände und Ausführungsformen, die für erfindungsgemäße Protease und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verfahren gilt.

Generell kann die Konfektionierung hierin beschriebener Mittel, insbesondere Geschirrspülmittel, in unterschiedlicher Weise erfolgen. Die Mittel können in fester oder flüssiger sowie als Kombination fester und flüssiger Angebotsformen vorliegen. Als feste Angebotsformen eignen sich insbesondere Pulver, Granulate, Extrudate, Kompaktate, insbesondere Tabletten. Die flüssigen Angebotsformen auf Basis von Wasser und/oder organischen Lösungsmitteln können verdickt, in Form von Gelen vorliegen. Die Mittel können in Form mehrphasiger Produkte konfektioniert werden. Die einzelnen Phasen solcher mehrphasiger Mittel können gleiche oder unterschiedliche Aggregatzustände aufweisen.

Die Mittel, insbesondere Geschirrspülmittel, können als Formkörper vorliegen. Um den Zerfall solcher vorgefertigter Formkörper zu erleichtern, ist es möglich, Desintegrationshilfsmittel, so genannte Tablettensprengmittel, in diese Mittel einzuarbeiten, um die Zerfallszeiten zu verkürzen. Unter Tablettensprengmitteln bzw. Zerfallsbeschleunigern werden Hilfsstoffe verstanden, die für den raschen Zerfall von Tabletten in Wasser oder anderen Medien und für die zügige Freisetzung der Wirkstoffe sorgen. Bevorzugt können Desintegrationshilfsmittel in Mengen von 0,5 bis 10 Gew.- %, vorzugsweise 3 bis 7 Gew.-% und insbesondere 4 bis 6 Gew.-%, jeweils bezogen auf das Gesamtgewicht des desintegrationshilfsmittelhaltigen Mittels, eingesetzt werden.

Die hierin beschriebenen Mittel, insbesondere Geschirrspülmittel, noch bevorzugter maschinellen Geschirrspülmittel, werden vorzugsweise zu Dosiereinheiten vorkonfektioniert. Diese Dosiereinheiten umfassen vorzugsweise die für einen Reinigungsgang notwendige Menge an reinigungsaktiven Substanzen. Bevorzugte Dosiereinheiten weisen ein Gewicht zwischen 12 und 30 g, bevorzugt zwischen 14 und 26 g und insbesondere zwischen 15 und 22 g auf. Das Volumen der vorgenannten Dosiereinheiten sowie deren Raumform sind mit besonderem Vorzug so gewählt, dass eine Dosierbarkeit der vorkonfektionierten Einheiten über die Dosierkammer einer Geschirrspülmaschine gewährleistet ist. Das Volumen der Dosiereinheit beträgt daher bevorzugt zwischen 10 und 35 ml, vorzugsweise zwischen 12 und 30 ml.

Die Mittel, insbesondere Geschirrspülmittel, insbesondere die vorgefertigten Dosiereinheiten weisen mit besonderem Vorzug eine wasserlösliche Umhüllung auf.

Die wasserlösliche Umhüllung wird vorzugsweise aus einem wasserlöslichen Folienmaterial, welches ausgewählt ist aus der Gruppe, bestehend aus Polymeren oder Polymergemischen, gebildet. Die Umhüllung kann aus einer oder aus zwei oder mehr Lagen aus dem wasserlöslichen Folienmaterial gebildet werden. Das wasserlösliche Folienmaterial der ersten Lage und der weiteren Lagen, falls vorhanden, kann gleich oder unterschiedlich sein. Besonders bevorzugt sind Folien, die z.B. zu Verpackungen wie Schläuchen oder Kissen verklebt und/oder versiegelt werden können, nachdem sie mit einem Mittel befüllt wurden.

Die wasserlösliche Verpackung kann eine oder mehr Kammern aufweisen. Das Mittel kann in einer oder mehreren Kammern, falls vorhanden, der wasserlöslichen Umhüllung enthalten sein. Die Menge an Mittel entspricht vorzugsweise der vollen oder halben Dosis, die für einen Spülgang benötigt wird.

Es ist bevorzugt, dass die wasserlösliche Umhüllung Polyvinylalkohol oder ein Polyvinylalkoholcopolymer enthält. Wasserlösliche Umhüllungen, die Polyvinylalkohol oder ein Polyvinylalkoholcopolymer enthalten, weisen eine gute Stabilität bei einer ausreichend hohen Wasserlöslichkeit, insbesondere Kaltwasserlöslichkeit, auf.

Geeignete wasserlösliche Folien zur Herstellung der wasserlöslichen Umhüllung basieren bevorzugt auf einem Polyvinylalkohol oder einem Polyvinylalkoholcopolymer, dessen Molekulargewicht im Bereich von 5.000 bis 1.000.000 g/mol, vorzugsweise von 20.000 bis 500.000 g/mol, besonders bevorzugt von 30.000 bis 100.000 g/mol und insbesondere von 40.000 bis 80.000 g/mol liegt.

Die Herstellung von Polyvinylalkohol geschieht üblicherweise durch Hydrolyse von Polyvinylacetat, da der direkte Syntheseweg nicht möglich ist. Ähnliches gilt für Polyvinylalkoholcopolymere, die aus entsprechend aus Polyvinylacetatcopolymeren hergestellt werden. Bevorzugt ist, wenn wenigstens eine Lage der wasserlöslichen Umhüllung einen Polyvinylalkohol umfasst, dessen Hydrolysegrad 70 bis 100 Mol-%, vorzugsweise 80 bis 90 Mol-%, besonders bevorzugt 81 bis 89 Mol-% und insbesondere 82 bis 88 Mol-% ausmacht.

Einem zur Herstellung der wasserlöslichen Umhüllung geeignetem Polyvinylalkoholenthaltendem Folienmaterial kann zusätzlich ein Polymer ausgewählt aus der Gruppe umfassend (Meth)Acrylsäure-haltige (Co)Polymere, Polyacrylamide, Oxazolin-Polymere, Polystyrolsulfonate, Polyurethane, Polyester, Polyether, Polymilchsäure oder Mischungen der vorstehenden Polymere zugesetzt sein. Ein bevorzugtes zusätzliches Polymer sind Polymilchsäuren.

Bevorzugte Polyvinylalkoholcopolymere umfassen neben Vinylalkohol Dicarbonsäuren als weitere Monomere. Geeignete Dicarbonsäuren sind Itaconsäure, Malonsäure, Bernsteinsäure und Mischungen daraus, wobei Itaconsäure bevorzugt ist.

Ebenfalls bevorzugte Polyvinylalkoholcopolymere umfassen neben Vinylalkohol eine ethylenisch ungesättigte Carbonsäure, deren Salz oder deren Ester. Besonders bevorzugt enthalten solche Polyvinylalkoholcopolymere neben Vinylalkohol Acrylsäure, Methacrylsäure, Acrylsäureester, Methacrylsäureester oder Mischungen daraus.

Es kann bevorzugt sein, dass das Folienmaterial weitere Zusatzstoffe enthält. Das Folienmaterial kann z.B. Weichmacher wie Dipropylenglycol, Ethylenglycol, Diethylenglycol, Propylenglycol, Glycerin, Sorbitol, Mannitol oder Mischungen daraus enthalten. Weitere Zusatzstoffe umfassen z.B. Freisetzungshilfen, Füllmittel, Vernetzungsmittel, Tenside, Antioxidationsmittel, UV-Absorber, Antiblockmittel, Antiklebemittel oder Mischungen daraus.

Geeignete wasserlösliche Folien zum Einsatz in den wasserlöslichen Umhüllungen der wasserlöslichen Verpackungen gemäß der Erfindung sind Folien, die von der Firma MonoSol LLC z.B. unter der Bezeichnung M8630, C8400 oder M8900 vertrieben werden. Andere geeignete Folien umfassen Folien mit der Bezeichnung Solublon^{®} PT, Solublon^{®} GA, Solublon^{®} KC oder Solublon^{®} KL von der Aicello Chemical Europe GmbH oder die Folien VF-HP von Kuraray.

Gemäß einer bevorzugten Ausführungsform ist das Mittel, insbesondere Geschirrspülmittel, von einer wasserlöslichen Folie eng umhüllt.

Die wasserlösliche Folie, welche bei der engen Umhüllung bevorzugt eingesetzt wird, umfasst besonders bevorzugt Polyvinylalkohol, wie oben beschrieben, wobei als Ausgangsdicke vorzugsweise eine Dicke von 10 µm bis 100 µm, insbesondere von 12 µm bis 60 µm, besonders bevorzugt von 15 µm bis 50 µm, vor allem von 20 µm bis 40 µm, insbesondere von 22 µm bis 35 µm verwendet wird.

Im Falle einer engen Umhüllung, ist jeweils eine Einmalportion des Reinigungsmittels umhüllt. Für die erfindungsgemäßen, umhüllten Reinigungsmitteleinmalportion ist es wichtig, dass die Umhüllung an jeder Stelle der Tabletten dicht an deren Oberfläche anliegt. Idealerweise steht die Umhüllung sogar unter Spannung, was jedoch nicht zwingend erforderlich ist. Dieses dichte Anliegen der Umhüllung ist förderlich für den Zerfall: Beim ersten Kontakt mit Wasser wird die Umhüllung an irgendeiner Stelle eine geringe Menge Wasser durchlassen, wobei sie sich zunächst überhaupt nicht zu lösen braucht. An dieser Stelle beginnt das in der Tablette enthaltene Sprengmittel zu quellen. Dies führt dazu, dass die Umhüllung nun infolge der Volumenzunahme der Tablette schlagartig aufreißt und die Tablette freigibt. Bei einer nicht enganliegenden Umhüllung funktioniert der hier beschrieben Mechanismus nicht, da die Tablette quellen kann, ohne dass die Umhüllung dadurch gesprengt würde. Dabei ist der Einsatz eines quellbaren Desintegrationsmittels einem gasentwickelnden System überlegen, da dessen sprengende Wirkung in jedem Fall zu einem Aufreißen der Umhüllung führt. Bei einem gasentwickelnden System kann die Sprengwirkung durch Entweichen des Gases aus einer Leckstelle der Umhüllung "verpuffen".

Erfindungsgemäße bevorzugte Reinigungsmitteleinmalportionen sind dadurch gekennzeichnet, dass der Abstand zwischen der Einmalportion und wasserlöslicher Umhüllung über die gesamte Fläche 0,1 bis 1000 µm, vorzugsweise 0,5 bis 500 µm, besonders bevorzugt 1 bis 250 µm und insbesondere 2,5 bis 100 µm, beträgt.

In einer bevorzugten Ausführungsform wird die Folienumhüllung zunächst lose um eine Reinigungsmitteleinmalportion gelegt und verschweißt und dann auf diese aufgeschrumpft, so dass ein enger Kontakt zwischen der Folienverpackung und dem Reinigungsmittelkonzentrat gegeben ist. Demzufolge sind erfindungsgemäße Reinigungsmitteleinmalportionen dadurch gekennzeichnet, dass die Umhüllung eine auf diese aufgeschrumpfte Folienverpackung ist.

Beispielsweise kann diese Umhüllung erfolgen, indem eine wasserlösliche Unterfolie auf eine Transportkette oder ein Form(en)werkzeug aufgelegt wird, dann eine oder mehrerer Wasch- oder Reinigungsmittelportion(en) auf die Unterfolie aufgelegt werden; anschließend eine wasserlösliche Oberfolie auf die Reinigungsmittelportion(en) auf der Unterfolie aufgelegt und diese dann auf der Unterfolie unter Einschluss der Reinigungsmittelportion(en) fixiert wird,

Alternativ kann dieser Schritt auch durch eine einsträngige Folie erfolgen, die dann als Schlauch um die Einmalportionen gelegt wird. Anschließend erfolgt ein Versiegeln und optionales Schneiden der Folien. Anschließend kann dann das Aufschrumpfen der Folie durch die Verwendung von Heißluft oder Infrarot-Strahlung, optional mit Andrücken, erfolgen.

Solche wasserlöslichen Umhüllungen sind auch in den Patentanmeldungen WO 2004/031338 A sowie WO 2003/099985 A, auf deren Offenbarung hiermit in vollem Umfang Bezug genommen wird, bereits beschrieben.

Die entsprechende Verwendung der erfindungsgemäßen Geschirrspülmittel ist ebenfalls Gegenstand der Erfindung. Ebenso betrifft die Erfindung ein Geschirrspülverfahren, insbesondere maschinelles oder manuelles Geschirrspülverfahren, bei welchem ein Geschirrspülmittel gemäß der Erfindung eingesetzt wird. Gegenstand der vorliegenden Anmeldung ist daher weiterhin ein Verfahren zur Reinigung von Geschirr, bei welchem das erfindungsgemäße Mittel verwendet wird. Wenn das erfindungsgemäße Geschirrspülmittel ein maschinelles Geschirrspülmittel ist, kann während des Durchlaufens eines Geschirrspülprogramms vor Beginn des Hauptspülgangs oder im Verlaufe des Hauptspülgangs das Mittel in den Innenraum einer Geschirrspülmaschine eindosiert werden. Die Eindosierung bzw. der Eintrag des erfindungsgemäßen Mittels in den Innenraum der Geschirrspülmaschine kann manuell erfolgen, vorzugsweise wird das Mittel jedoch mittels der Dosierkammer in den Innenraum der Geschirrspülmaschine dosiert.

Da erfindungsgemäße Proteasen natürlicherweise bereits eine hydrolytische Aktivität besitzen und diese auch in Medien entfalten, die sonst keine Reinigungskraft besitzen wie z.B. in bloßem Puffer, kann ein einzelner und/oder der einzige Schritt eines solchen Verfahrens darin bestehen, dass als einzige reinigungsaktive Komponente eine erfindungsgemäße Protease mit der Anschmutzung in Kontakt gebracht wird, bevorzugt in einer Pufferlösung oder in Wasser. Dies stellt eine weitere Ausführungsform dieses Erfindungsgegenstandes dar.

Alternative Ausführungsformen dieses Erfindungsgegenstandes stellen auch Verfahren zur Behandlung von Textilrohstoffen oder zur Textilpflege dar, bei denen in wenigstens einem Verfahrensschritt eine erfindungsgemäße Protease aktiv wird. Hierunter sind Verfahren für Textilrohstoffe, Fasern oder Textilien mit natürlichen Bestandteilen bevorzugt, und ganz besonders für solche mit Wolle oder Seide.

Schließlich erfasst die Erfindung auch die Verwendung der hierin beschriebenen Proteasen in Wasch- oder Reinigungsmitteln, z.B. wie oben beschrieben, zur (verbesserten) Entfernung von proteinhaltigen Anschmutzungen, z.B. von Textilien oder harten Oberflächen.

Alle Sachverhalte, Gegenstände und Ausführungsformen, die für erfindungsgemäße Protease und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehende erfindungsgemäße Verwendung gilt.

### Bevorzugte Ausführungsformen

1. Protease umfassend eine Aminosäuresequenz, die mindestens 70% Sequenzidentität mit der in SEQ ID NO:1 angegebenen Aminosäuresequenz über deren Gesamtlänge aufweist und die eine Aminosäuresubstitution an mindestens einer der Positionen, die den Positionen Q12, I43, M122, D127, N154, T156, G160, M211, P212 oder A222, jeweils bezogen auf die Nummerierung gemäß SEQ ID NO:1, entsprechen, aufweist.
2. Protease nach Anspruch 1, wobei die mindestens eine Aminosäuresubstitution aus der Gruppe bestehend aus Q12L, I43V, M122L, D127P, N154S, T156A, G160S, M211N, M211L, P212D, P212H oder A222S, jeweils bezogen auf die Nummerierung gemäß SEQ ID NO:1, ausgewählt ist.
3. Protease nach Anspruch 1 oder 2, wobei die Protease eine der folgenden Aminosäuresubstitutionsvarianten, jeweils bezogen auf die Nummerierung gemäß SEQ ID NO:1, aufweist:
   (i) I43V;
   (ii) M122L, N154S und T156A;
   (iii) M211N und P212D;
   (iv) M211L und P212D;
   (v) G160S;
   (vi) D127P, M211L und P212D;
   (vii) P212H; oder
   (viii) Q12L, M122L und A222S.
4. Protease, dadurch gekennzeichnet, dass
   (a) sie aus einer Protease nach Anspruch 1-3 als Ausgangsmolekül erhältlich ist durch ein- oder mehrfache konservative Aminosäuresubstitution, wobei die Protease mindestens eine der Aminosäuresubstitutionen Q12L, I43V, M122L, D127P, N154S, T156A, G160S, M211N, M211L, P212D, P212H oder A222S an den Positionen, die den Positionen 12, 43, 122, 127, 154, 156, 160, 211, 212 und 222 gemäß SEQ ID NO:1 entsprechen, aufweist; und/oder
   (b) sie aus einer Protease nach Anspruch 1-3 als Ausgangsmolekül erhältlich ist durch Fragmentierung, Deletions-, Insertions- oder Substitutionsmutagenese und eine Aminosäuresequenz umfasst, die über eine Länge von mindestens 190, 200, 210, 220, 230, 240, 250, 260 oder 269 zusammenhängenden Aminosäuren mit dem Ausgangsmolekül übereinstimmt, wobei die Protease mindestens eine der Aminosäuresubstitutionen Q12L, I43V, M122L, D127P, N154S, T156A, G160S, M211N, M211L, P212D, P212H oder A222S an den Positionen, die den Positionen 12, 43, 122, 127, 154, 156, 160, 211, 212 und 222 gemäß SEQ ID NO:1 entsprechen, umfasst.
5. Verfahren zur Herstellung einer Protease umfassend das Substituieren einer Aminosäure an mindestens einer der Positionen, die Positionen 12, 43, 122, 127, 154, 156, 160, 211, 212 und 222 in SEQ ID NO:1 entsprechen, in einer Ausgangsprotease, die mindestens 70% Sequenzidentität zu der in SEQ ID NO:1 angegebenen Aminosäuresequenz über deren Gesamtlänge aufweist, vorzugsweise derart, dass die Protease an mindestens einer Position die Aminosäuresubstitution Q12L, I43V, M122L, D127P, N154S, T156A, G160S, M211N, M211L, P212D, P212H oder A222S umfasst.
6. Verfahren nach Anspruch 5, ferner umfassend einen oder beide der folgenden Verfahrensschritte:
   (a) Substituieren mindestens einer Aminosäure durch ein- oder mehrfache konservative Aminosäuresubstitution, wobei die Protease mindestens eine der Aminosäuresubstitutionen Q12L, I43V, M122L, D127P, N154S, T156A, G160S, M211N, M211L, P212D, P212H oder A222S an den Positionen, die den Positionen 12, 43, 122, 127, 154, 156, 160, 211, 212 und 222 gemäß SEQ ID NO:1 entsprechen, umfasst;
   (b) Veränderung der Aminosäuresequenz durch Fragmentierung, Deletions-, Insertions- oder Substitutionsmutagenese derart, dass die Protease eine Aminosäuresequenz umfasst, die über eine Länge von mindestens 190, 200, 210, 220, 230, 240, 250, 260 oder 269 zusammenhängenden Aminosäuren mit dem Ausgangsmolekül übereinstimmt, wobei die Protease mindestens eine der Aminosäuresubstitutionen Q12L, I43V, M122L, D127P, N154S, T156A, G160S, M211N, M211L, P212D, P212H oder A222S an den Positionen, die den Positionen 12, 43, 122, 127, 154, 156, 160, 211, 212 und 222 gemäß SEQ ID NO:1 entsprechen, umfasst.
7. Nukleinsäure kodierend für eine Protease nach einem der Ansprüche 1-4 oder kodierend für eine nach einem Verfahren nach einem der Ansprüche 5 oder 6 erhältliche Protease.
8. Vektor enthaltend eine Nukleinsäure nach Anspruch 7, insbesondere ein Klonierungsvektor oder ein Expressionsvektor.
9. Nicht-menschliche Wirtszelle, die eine Nukleinsäure nach Anspruch 7 oder einen Vektor nach Anspruch 8 beinhaltet, oder die eine Protease nach einem der Ansprüche 1-4 beinhaltet, oder die eine nach einem Verfahren der Ansprüche 5 oder 6 erhältliche Protease beinhaltet.
10. Verfahren zur Herstellung einer Protease umfassend
   a) Kultivieren einer Wirtszelle gemäß Anspruch 9; und
   b) Isolieren der Protease aus dem Kulturmedium oder aus der Wirtszelle.
11. Mittel, insbesondere ein Wasch- oder Reinigungsmittel, dadurch gekennzeichnet, dass es mindestens eine Protease nach einem der Ansprüche 1-4 oder eine nach einem Verfahren der Ansprüche 5 oder 6 erhältliche Protease enthält.
12. Mittel nach Anspruch 11, dadurch gekennzeichnet, dass das Mittel ein Geschirrspülmittel, bevorzugt ein automatisches Geschirrspülmittel, ist.
13. Verfahren zur Reinigung von Textilien oder harten Oberflächen, dadurch gekennzeichnet, dass in mindestens einem Verfahrensschritt ein Mittel nach Anspruch 11 oder 12 angewendet wird, oder dass in mindestens einem Verfahrensschritt eine Protease nach einem der Ansprüche 1-4 oder eine nach einem Verfahren der Ansprüche 5 oder 6 erhältliche Protease angewendet wird.
14. Verwendung einer Protease nach einem der Ansprüche 1-4 oder einer nach einem Verfahren der Ansprüche 5 oder 6 erhältlichen Protease in einem Wasch- oder Reinigungsmittel zur Entfernung von peptid- oder proteinhaltigen Anschmutzungen.

### Beispiele

### Übersicht über die Mutationen der Varianten:

| **Variante** | **Sequenz** | | | | **SEQ ID NO:** |
|---|---|---|---|---|---|
| Mutante 1 | I43V | | | | 2 |
| Mutante 2 | M122L | N154S | T156A | | 3 |
| Mutante 3 | M211N | P212D | | | 4 |
| Mutante 4 | M211L | P212D | | | 5 |
| Mutante 5 | G160S | | | | 6 |
| Mutante 6 | D127P | M211L | P212D | | 7 |
| Mutante 7 | P212H | | | | 8 |
| Mutante 8 | Q12L | M122L | A222S | | 9 |

### Aktivitätsbestimmung der Protease

Die Proteaseaktivität wird in einem diskontinuierlichen Assay bestimmt, in dem Casein als Substrat verwendet wird. Die Endkonzentration der Substrat-Lösung beträgt 12 mg/ml Casein (hergestellt nach Hammarsten; Merck, Darmstadt, #2242) und 30 mM Tris in synthetischem Leitungswasser. Synthetisches Leitungswasser ist eine Lösung von 0,029% (w/v) CaCl₂.2H₂O, 0,014% (w/v) MgCl₂.6H₂O und 0,021% (w/v) NaHCO₃ mit 15W°dH (deutscher Härte). Die Substrat-Lösung wird auf 70°C erhitzt und ihr pH wird auf 8,5 bei 50°C durch Verwendung von 0,1 N NaOH eingestellt. Die Protease-Lösung wird hergestellt durch Zugabe von 2% (w/v) wasserfreiem Pentanatriumtripolyphosphat in synthetisches Leistungswasser und Einstellung auf pH 8,5 mit Salzsäure. Zu 600 µl der Casein-Lösung wird 200 µl der Enzym-Lösung gegeben. Das Gemisch wird bei 50°C für 15 Minuten inkubiert. Die Reaktion wird durch die Zugabe von 600 µl von 0,44 M Trichloressigsäure (TCA), 0,22 M Natriumacetat in 3% (w/v) beendet. Nach einem Kühlungsschritt von 15 Minuten auf Eis wird das TCA-unlösliche Protein durch Zentrifugation entfernt. 900 µl der verbleibenden Lösung werden mit 300 µl von 2 N NaOH gemischt und die Absorption dieses Gemisches, das TCA-lösliche Proteine enthält, wird bei 290 nm gemessen. Kontrollwerte werden erzeugt durch die Zugabe von 600 µl TCA-Lösung zu 600 µl Casein-Lösung gefolgt von einer Zugabe von 200 µl Enzymlösung. Eine Protease-Lösung, die unter diesen Bedingungen eine Absorptionsänderung von 0,500 OD bei 290 nm bewirkt, hat nach vorliegender Bezeichnung eine Aktivität von 10 HPE pro ml.

### Untersuchung der Varianten in einer Geschirrspülmittelmatrix

Es wurde ein Phosphat-freies handelsübliches automatisches Geschirrspülmittel in Form einer Geschirrspülmitteltablette verwendet. Das Tablettengewicht lag bei 19 g. Die Geschirrspülmittelmatrix besaß die folgende Zusammensetzung:

| Rohstoff | P-frei Formelbereiche | |
|---|---|---|
| | Gesamt (Gew.-%) | g/Job |
| Na-Citrat | 15,00 - 20,00 | 3,00 - 4,00 |
| Phosphonat (HEDP) | 2,50 - 7,50 | 0,50 - 1,50 |
| MGDA | 0,00 - 25,00 | 0,00 - 1,50 |
| Na-Disilikat | 5,00 - 35,00 | 1,00 - 7,00 |
| Soda | 12,50 - 25,00 | 2,50 - 5,00 |
| Na-Percarbonat | 10,00 - 15,00 | 2,00 - 3,00 |
| Bleichkatalysator (Mn-basiert) | 0,02 - 0,50 | 0,003 - 0,10 |
| TAED | 2,00 - 3,00 | 0,40 - 0,60 |
| Nio Tensid 20-40EO end-cap | 2,50 - 10,00 | 0,50 - 2,00 |
| Polycarboxylat | 5,00 - 10,00 | 1,00 - 2,00 |
| Kationisches Co-Polymer | 0,25 - 0,75 | 0,05 - 0,15 |
| Quervernetztes PVP | 0,00 - 1,50 | 0,00 - 0,30 |
| Protease | 1,50 - 5,00 | 0,30 - 1,00 |
| Amylase | 0,50 - 3,00 | 0,10 - 0,60 |
| Benzotriazol (Silberschutz) | 0,00 - 0,50 | 0,00 - 0,10 |
| Parfüm | 0,05 - 0,15 | 0,01 - 0,03 |
| Farbstofflösung | 0,00 - 1,00 | 0,00 - 0,20 |
| Zn-Acetat | 0,10 - 0,30 | 0,02 - 0,06 |
| Na-Sulfat | 0,00 - 25,00 | 0,00 - 5,00 |
| Wasser | 0,00 - 1,50 | 0,00 - 0,30 |
| pH-Stellmittel (Citronensäure) | 1,00 - 1,50 | 0,20 - 0,30 |
| Prozessmittel | 0,00 - 5,00 | 0,00 - 1,00 |
| | 57,92 - 196,20 | 11,6 - 39,24 |

| | | |
|---|---|---|
| berechnet auf 20 g Tablette (die Tablette kann auch 17 - 20g wiegen) | | |

Die Reinigungsleistung beschreibt das Vermögen eines Geschirrspülmittels, insbesondere eines maschinellen Geschirrspülmittels, eine vorhandene Anschmutzung teilweise oder vollständig zu entfernen. Es wurde die Reinigungsleistung des Mittels an verschiedenen hartnäckigen Anschmutzungen getestet. Dem eingesetzten Mittel waren jeweils die erfindungsgemäßen Proteasevarianten zugesetzt.

Das Geschirrspülverfahren wurde in der Spülmaschine Miele GSL (Programm: 45°C, 8 min Haltezeit, Programmdauer 57 min, Wasserhärte 21° deutscher Härte) nach IKW Standard durchgeführt. Die Geschirrspülmitteltablette wurde vor Beginn des Reinigungsprogramms in die Dosiervorrichtung gegeben.

Die Auswertung der Reinigungsleistung erfolgte visuell gemäß einer Skala von 1 bis 10, wobei der Wert 10 die beste Reinigungsleistung repräsentiert (kein erkennbarer Rückstand). Es erfolgen 3 Wiederholungen mit jeweils 6 internen Replikationen pro Maschine. Die angegebenen Ergebnisse sind der Mittelwert der Mehrfachbestimmung.

Es ergaben sich folgende delta-Werte im Vergleich zur Wildtyp-Protease (SEQ ID NO:1):

| | Tee (Assam) | Tee (BOP) | Hackfleisch | Spaghetti | Crème Brûlée |
|---|---|---|---|---|---|
| Variante 1 | n.d. | n.d. | 1,4 | 1,1 | 0,4 |
| Variante 2 | 0,8 | 0,8 | 1,6 | 0,6 | n.d. |
| Variante 3 | 0,6 | 0,6 | 0,6 | 1,2 | 0,4 |
| Variante 4 | 1,0 | 0,9 | 0,9 | 1,1 | 1,2 |
| Variante 5 | 0,7 | 0,1 | 0,7 | 1,3 | n.d. |
| Variante 6 | 1,2 | 0,7 | n.d. | 0,9 | n.d. |
| Variante 7 | n.d. | n.d. | n.d. | 1,0 | n.d. |
| Variante 8 | 0,6 | 1,0 | n.d. | n.d. | n.d. |

Wie zu erkennen ist, führt die Verwendung der Proteasevarianten zu einer Verbesserung der Reinigungsleistung. Insbesondere Varianten 4, 6 und 8 führen zu einer Verbesserung auf Tee-Anschmutzungen. Varianten 1 und 2 zeigen besondere Vorteile auf Hackfleisch und Varianten 1, 3, 4, 5 und 7 auf Spaghetti. Weiterhin ist bei Variante 4 ein besonderer Vorteil auf Crème Brûlée zu beobachten.

## Patentansprüche

1. Protease umfassend eine Aminosäuresequenz, die mindestens 70% Sequenzidentität mit der in SEQ ID NO:1 angegebenen Aminosäuresequenz über deren Gesamtlänge aufweist und die eine Aminosäuresubstitution an mindestens einer der Positionen, die den Positionen P212, Q12, I43, M122, D127, N154, T156, G160, M211 oder A222, jeweils bezogen auf die Nummerierung gemäß SEQ ID NO:1, entsprechen, aufweist.

2. Protease nach Anspruch 1, wobei die mindestens eine Aminosäuresubstitution aus der Gruppe bestehend aus P212D, P212H, Q12L, I43V, M122L, D127P, N154S, T156A, G160S, M211N, M211L oder A222S, jeweils bezogen auf die Nummerierung gemäß SEQ ID NO:1, ausgewählt ist.

3. Protease nach Anspruch 1 oder 2, wobei die Protease eine der folgenden Aminosäuresubstitutionsvarianten, jeweils bezogen auf die Nummerierung gemäß SEQ ID NO:1, aufweist:
(i) I43V;
(ii) M122L, N154S und T156A;
(iii) M211N und P212D;
(iv) M211L und P212D;
(v) G160S;
(vi) D127P, M211L und P212D;
(vii) P212H; oder
(viii) Q12L, M122L und A222S.

4. Automatisches Geschirrspülmittel, umfassend
(A) mindestens eine Protease, die proteolytische Aktivität aufweist und eine Aminosäuresequenz, die mindestens 70% Sequenzidentität mit der in SEQ ID NO:1 angegebenen Aminosäuresequenz über deren Gesamtlänge aufweist und die eine Aminosäuresubstitution an mindestens einer der Positionen, die den Positionen P212, Q12, I43, M122, D127, N154, T156, G160, M211 oder A222, jeweils bezogen auf die Nummerierung gemäß SEQ ID NO:1, entsprechen, aufweist, und
(B) mindestens ein Tensid und/oder
(C) mindestens einen Builder (Gerüststoff) und/oder
(D) mindestens eine Persauerstoffverbindung und/oder
(E) mindestens einen Bleichaktivator kann.

5. Mittel nach Anspruch 4, wobei die Protease mindestens eine Aminosäuresubstitution aufweist, die aus der Gruppe bestehend aus P212D, P212H, Q12L, I43V, M122L, D127P, N154S, T156A, G160S, M211N, M211L oder A222S, jeweils bezogen auf die Nummerierung gemäß SEQ ID NO:1, ausgewählt ist.

6. Mittel nach Anspruch 4 oder 5, wobei die Protease eine der folgenden Aminosäuresubstitutionsvarianten, jeweils bezogen auf die Nummerierung gemäß SEQ ID NO:1, aufweist:
(i) I43V;
(ii) M122L, N154S und T156A;
(iii) M211N und P212D;
(iv) M211L und P212D;
(v) G160S;
(vi) D127P, M211L und P212D;
(vii) P212H; oder
(viii) Q12L, M122L und A222S.

7. Mittel nach einem der Ansprüche 4 bis 6, wobei das Mittel keine Borsäure enthält.

8. Verfahren zur Reinigung von harten Oberflächen, insbesondere Geschirr, **dadurch gekennzeichnet, dass** in mindestens einem Verfahrensschritt ein Mittel nach einem der Ansprüche 4 bis 7 angewendet wird, oder dass in mindestens einem Verfahrensschritt eine Protease nach einem der Ansprüche 1 bis 3 angewendet wird.

9. Verfahren nach Anspruch 8, wobei in mindestens einem Verfahrensschritt ein Mittel nach einem der Ansprüche 4 bis 7 angewendet wird, oder dass in mindestens einem Verfahrensschritt eine Protease nach einem der Ansprüche 1 bis 3 katalytisch aktiv wird, wobei die Protease in einer Menge von 40 µg bis 4 g, vorzugsweise von 50 µg bis 3 g, besonders bevorzugt von 100 µg bis 2 g und ganz besonders bevorzugt von 200 µg bis 1 g eingesetzt wird.

10. Verfahren nach Anspruch 8 oder 9, wobei das Verfahren bei einer Temperatur von 0-100°C, bevorzugt 10-70°C, weiter bevorzugt 30-50°C und am meisten bevorzugt bei 45°C durchgeführt wird.

11. Verwendung einer Protease nach einem der Ansprüche 1 bis 3 in einem automatischen Geschirrspülmittel zur Entfernung von peptid- oder proteinhaltigen Anschmutzungen von Oberflächen, insbesondere Geschirr.

12. Verwendung eines Mittels nach einem der Ansprüche 4 bis 7 zur Entfernung von peptid- oder proteinhaltigen Anschmutzungen von Oberflächen, insbesondere Geschirr.

13. Verwendung einer Protease nach einem der Ansprüche 1 bis 3 in einem Mittel nach einem der Ansprüche 4 bis 7 zur Entfernung von peptid- oder proteinhaltigen Anschmutzungen von Oberflächen, insbesondere Geschirr.
